# EUROPEAN PATENT SPECIFICATION

(11) **EP 1 926 504 B1**
(45) Date of publication and mention of the grant of the patent: **13.01.2010**
(21) Application number: 06803981.7
(22) Date of filing: 21.09.2006
(51) Int. Cl.: A61L 24/08

(54) **IN SITU OCCLUDING COMPOSITIONS NCLUDING NATURAL BIODEGRADABLE POLYSACCHARIDES**
IN SITU OKKLUSIONSZUSAMMENSETZUNGEN MIT NATÜRLICHEN BIOLOGISCH ABBAUBAREN POLYSACCHARIDEN
OCCLUSION IN SITU UTILISANT DES POLYSACCHARIDES BIODEGRADABLES NATURELS

(30) Priority: 21.09.2005 US 719466 P; 10.04.2006 US 791086 P
(43) Date of publication of application: 04.06.2008
(73) Proprietor: SurModics, Inc., Eden Prairie, MN 55344 (US)
(72) Inventor: CHUDZIK, Stephen, J., St. Paul, Minnesota 55101 (US); CHINN, Joseph, A., Shakopee, Minnesota 55379 (US); SWAN, Dale, G., St. Louis Park, Minnesota 55426 (US); BURKSTRAND, Michael, J., Richfield, Minnesota 55423 (US); DUQUETTE, Peter, H., Edina, Minnesota 55439 (US)
(74) Representative: Stoner, Gerard Patrick
(86) International application number: PCT/US2006/036812
(87) International publication number: WO 2007/035865

(56) References cited:
- WO-A-93/09176
- WO-A-2005/034875
- US-A1- 2003 077 242
- US-A1- 2005 133 046
- US-A1- 2005 136 091

## Description

### Technical Field

The present invention relates to *in situ* formed biodegradable occlusions comprising a natural biodegradable polymeric material.

### Background

Embolic compositions can be used to form matrices *in situ* and coatings having embolic properties. Embolic compositions can be used to control fluid movement by the formation of an embolic mass by itself or in association with a surface. Such compositions are useful for sealing endoleaks in aneurysms, filling aneurysm sacs, treating arteriovenous fistulas and arteriovenous malformations, occluding blood vessels, and occluding fallopian tubes.

Embolic compositions can be delivered to a desired location of the body and then polymerized at that location to provide an *in situ*-formed hydrogel. Many non-biodegradable macromer systems have been described and proposed for use in the body as embolic agents. See, for example, U.S. Patent Nos. 5,410,016, 5,626,863, and 6,676,971.

Existing macromer technologies, however, are less than ideal. Many macromer systems are based on non-biodegradable polymer systems, such as poly(vinylalcohol) (PVA). Matrices formed from these macromer systems generally are not capable of being degraded and reabsorbed by the body. Since aneurysms place pressure on tissue or organs that are in contact by the aneurysm, the embolic occlusions formed from non-biodegradable materials generally will not allow the aneurysm to shrink and relieve pressure on the adjacent tissue.

Polyglycolide materials have also been extensively used for the preparation of articles that are used *in vivo.* Polyglycolides are pH sensitive and are degraded by hydrolysis. This can present stability concerns. Also, articles formed from polyglycolides exhibit bulk degradation, rather than surface degradation. *In vivo,* this may result in portions of the degrading article dislodging and being relocated to a different portion of the body via body fluids, which may cause problems at this secondary site. Furthermore, polyglycolide materials do not bond well to tissue. Lack of adhesion can lead to localized areas of undesired flow at the site of embolic mass formation, such as in an aneurysm.

Polyglycolides also degrade into acidic compounds. These acidic degradation products have been reported to be associated with undesirable non-infective inflammatory responses. These acidic degradation products may also have an effect on the function of polypeptides by interacting with basic residues on portions of the polypeptide. Such interactions would be undesirable if the biodegradable article is associated with a bioactivity provided by the polypeptide.

Embolic compositions can also be in the form of polymeric coatings which can provide a sealant function to medical articles. Biodegradable sealant compositions have been used on articles having porous surfaces, such as fabrics associated with implantable medical articles. The sealant coating initially renders the porous surface impermeable to fluids for a period of time. However, as the sealant materials degrade and are resorbed by the body, cells involved in tissue repair infiltrate the porous material and replace the sealant materials. Thus, newly formed tissue replaces the original function of the coated sealant over a period of time.

Animal-derived sealant materials such as collagen and gelatin are commonly used to coat textile grafts. These materials can be resorbed *in vivo.* The blood clotting protein fibrin has also been utilized as a sealant material. Despite their uses, there are drawbacks and concerns with using these types of sealant materials. One particular problem is that it is difficult to produce consistent sealant compositions from these animal sources due to batch-to-batch variations inherent in their production.

In many cases the collagen used in sealant technologies is obtained from non-human animal sources, such as bovine sources. In these cases there is the possibility that bovine collagen preparations may contain unwanted contaminants that are undesirable for introduction into a human subject. One example of an unwanted contaminant is the prionic particles that cause Bovine Spongiform Encephalopathy (BSE).

BSE, also termed Mad Cow Disease, is one of a group of progressive neurological diseases called transmissible spongiform encephalopathies, or TSEs (named for deteriorated areas of the brain that look like sponges). Various forms of TSE have been reported, including scrapie in sheep and chronic wasting disease in elk and mule deer. It is generally believed that the use of recycled animal parts led to the cross-species contamination of scrapie in sheep to mad cow disease, and the ingestion of contaminated beef and bovine products led to the human variant of this disease, Creutzfeldt-Jakob Disease (CJD).

Additional concerns are that preparations from animal sources may provide other unwanted contaminants, such as antigenic factors. These antigenic factors may promote a localized immune response in the vicinity of the implanted article and foul its function. These factors may also cause infection as well as local inflammation.

While synthetic materials can be used in the preparation of sealant compositions, these synthetic materials have the potential of degrading into non-naturally occurring products. These non-naturally occurring products have the potential to be at least partially toxic to the organism or immunogenic and cause inflammation, as well as infection, at or around the site of implantation.

The composition according to the invention comprises a natural biodegradable polysaccharide comprising pendent polymerisable groups and a first member of a redox pair,
for use in a method of medical treatment which comprises forming a biodegradable occlusion at a target site within the body by delivering the composition to the target site and contacting the composition at the target site with a second member of the redox pair where the redox pair initiates reaction of the pendent polymerisable groups of the natural biodegradable polysaccharide to form the biodegradable occlusion.

Another aspect (claim 21) is a kit for forming a biodegradable occlusion at a target site within the body, comprising
a first composition for delivery to the target site, comprising natural biodegradable polysaccharide comprising pendent polymerisable groups and a first member of a redox pair, and
a second composition for delivery to the target site, comprising natural biodegradable polysaccharide comprising pendent polymerisable groups and the second member of the redox pair,
whereby in use, on contacting the first and second compositions, the resulting redox pair initiates polymerisation of the polymerisable groups to form the biodegradable occlusion,

A further aspect (claim 22) is a kit for forming a biodegradable occlusion at a target site within a body, the kit comprising:
a natural biodegradable polysaccharide comprising a pendent polymerisable group;
a first member of a redox pair;
an article configured to be delivered to the target site, and
a second member of the redox pair.

In some aspects, the composition can include a radiopacifying agent. For example, a radiopacifying agent comprising iodine can be associated with the natural biodegradable polysaccharide. Iodine is thought to complex with the polysaccharide (such as amylose or maltodextrin), which acts as an iodine-binding compound. This can advantageously improve medical procedures wherein an imaging step is performed. For example, biodegradable occlusions formed from natural biodegradable polysaccharides that also include iodine may be more readily visualized based on the density of iodine associated with the occlusion.

In the formation of the biodegradable occlusion, a plurality of natural biodegradable polysaccharides are crosslinked to each other via coupling groups that are pendent from the natural biodegradable polysaccharide (i.e., one or more coupling groups are chemically bonded to the polysaccharide). The coupling group on the natural biodegradable polysaccharide is a polymerizable group. In a free radical polymerization reaction the polymerizable group can crosslink natural biodegradable polysaccharides together in the composition, thereby forming a natural biodegradable polysaccharide matrix *in-vivo.*

The natural biodegradable polysaccharides described herein are non-synthetic polysaccharides that can be associated with each other to form a matrix, which can be used as an *in-situ* formed matrix. The natural biodegradable polysaccharides can also be enzymatically degraded, but offer the advantage of being generally non-enzymatically hydrolytically stable. This is particularly advantageous for bioactive agent delivery, as in some aspects the invention provides articles capable of releasing the bioactive agent under conditions of enzyme-mediated degradation, but not by diffusion. Therefore, the kinetics of bioactive agent release from the articles of the invention are fundamentally different than those prepared from synthetic biodegradable materials, such as poly(lactides).

Natural biodegradable polysaccharides include polysaccharide and/or polysaccharide derivatives that are obtained from natural sources, such as plants or animals. Exemplary natural biodegradable polysaccharides include maltodextrin, amylose, cyclodextrin, polyalditol, hyaluronic acid, dextran, heparin, chondroitin sulfate, dermatan sulfate, heparan sulfate, keratan sulfate, dextran, dextran sulfate, pentosan polysulfate, and chitosan. Preferred polysaccharides are low molecular weight polymers that have little or no branching, such as those that are derived from and/or found in starch preparations, for example, amylose and maltodextrin.

Because of the particular utility of the amylose and maltodextrin polymers, in some aspects natural biodegradable polysaccharides are used that have an average molecular weight of 500,000 Da or less, 250,000 Da or less, 100,000 Da or less, or 50,000 Da or less. In some aspects the natural biodegradable polysaccharides have an average molecular weight of 500 Da or greater. In some aspects the natural biodegradable polysaccharides have an average molecular weight in the range of about 1000 Da to about 10,000 Da. Natural biodegradable polysaccharides of particular molecular weights can be obtained commercially or can be prepared, for example, by acid hydrolysis and/or enzymatic degradation of a natural biodegradable polysaccharide preparation, such as starch. The decision of using natural biodegradable polysaccharides of a particular size range may depend on factors such as the physical characteristics of the composition (e.g., viscosity), the desired rate of degradation of the matrix, the presence of other optional moieties in the composition (for example, bioactive agents, etc.), etc.

The natural biodegradable polysaccharides that are used in accordance with the methods and compositions of the invention are readily available at a low cost and/or can be prepared easily using established techniques.

The use of natural biodegradable polysaccharides, such as maltodextrin or amylose, provides many advantages when used in a composition for the formation of an article, such as one that can be used *in vivo.* Degradation of a natural biodegradable polysaccharide-containing article can result in the release of, for example, naturally occurring mono- or disaccharides, such as glucose, which are common serum components. Furthermore, the use of natural biodegradable polysaccharides that degrade into common serum components, such as glucose, can be viewed as more acceptable than the use of synthetic biodegradable polysaccharides that degrade into non-natural compounds, or compounds that are found at very low concentrations in the body.

In some aspects of the invention, this advantageous feature is reflected in the use of natural biodegradable polysaccharides which are non-animal derived, such as amylose and maltodextrin, and that degrade into products that present little or no immunogenic or toxic risk to the individual. The invention provides improved, cost-efficient, natural biodegradable polysaccharide compositions for articles that can be used in a variety of medical treatments.

Another advantage of the invention is that the natural biodegradable polysaccharides-containing matrices are more resistant to hydrolytic degradation than other matrices prepared from biodegradable polymers, such as poly(lactides). Degradation of the natural biodegradable polysaccharides of the invention are primarily enzyme-mediated, with minimal or no hydrolysis of the natural biodegradable polysaccharide occurring under ambient conditions. This allows the natural biodegradable polysaccharides to remain substantially stable (for example, resistant to degradation) prior to forming a matrix *in vivo.* Other biodegradable polymers such as poly(lactide) or poly(lactide-co-glycolide) are subject to hydrolysis even at relatively neutral pH ranges (e.g., pH 6.5 to 7.5) and therefore do not offer this advantage.

Therefore, the invention includes natural biodegradable polysaccharide-containing compositions that have the advantage of providing stability in the presence of an aqueous environment.

In one aspect, the invention provides a shelf-stable composition for preparing a biodegradable occlusion at a target site within the body, the shelf stable composition comprising a natural biodegradable polysaccharide comprising pendent polymerisable groups and a first member of a redox pair. These compositions could be obtained or prepared, according to the details provided herein, and then stored for a period of time before the composition is used, without significant degradation of the natural biodegradable polysaccharide occurring during storage. Optionally, one or more bioactive agents and/or microparticles can be added before or after storage of the composition.

In a related aspect, the invention also provides the advantage of being able to perform methods wherein the natural biodegradable polysaccharide is subject to exposure to an aqueous solution without risking significant degradation of the natural biodegradable polysaccharide. For example, the natural biodegradable polysaccharide may be contacted with an aqueous solution in a synthetic or post-synthetic step, including addition synthesis reactions and purification steps.

Degradation of the natural biodegradable polysaccharide-containing matrix may commence when placed in contact with a body fluid, which may include natural biodegradable polysaccharide-degrading enzymes, such as carbohydrases.

The invention also provides a useful way to deliver larger hydrophilic bioactive agents, such as polypeptides, nucleic acids, and polysaccharides, as well as viral particles and cells from the biodegradable article. Comparatively, the use of non-degrading drug delivery matrices may not be useful for the delivery of these larger bioactive agents if they are too large to diffuse out of the matrix. However, according to some aspects of the invention, an article that includes a matrix of the natural biodegradable polysaccharide having a bioactive agent is formed in the body, and as the matrix degrades the bioactive agent is gradually released from the matrix. In one aspect of the invention, the bioactive agent has a molecular weight of about 10,000 Da or greater.

In some aspects, the invention provides a drug-releasing biodegradable matrix comprising (i) a natural biodegradable polysaccharide, preferably selected from amylose and maltodextrin, comprising an ethylenically unsaturated group, (ii) an initiator, and (iii) a bioactive agent selected from the group of polypeptides, polynucleotides, and polysaccharides.

In another aspect of the invention, the natural biodegradable polysaccharide is modified with a hydrophobic moiety in order to provide a biodegradable matrix having hydrophobic properties. Therefore, a biodegradable occlusion can be formed from natural biodegradable polysaccharide comprising one or more pendent polymerisable groups and one or more pendent hydrophobic moieties. Exemplary hydrophobic moieties include fatty acids and derivatives thereof, and C₂-C₁₈ alkyl chains.

Therefore, in some aspects of the invention, modification of the natural biodegradable polysaccharide allows for preparation of occlusions that are biodegradable and that can release a hydrophobic bioactive agent.

In other aspects, the hydrophobic moiety pendent from the natural biodegradable has properties of a bioactive agent. Upon degradation of the matrix, the hydrophobic moiety can be hydrolyzed from the natural biodegradable polymer and released to provide a therapeutic effect. One example of a therapeutically useful hydrophobic moiety is butyric acid.

In yet another aspect, the invention provides improved stability of a bioactive agent that is delivered from an matrix formed from natural biodegradable non-reducing polysaccharides. The non-reducing polysaccharide can provide an inert matrix and thereby improve the stability of sensitive bioactive agents, such as proteins and enzymes. The occlusion can include a matrix having a plurality of natural biodegradable non-reducing polysaccharides along with a bioactive agent, such as a polypeptide. An exemplary non-reducing polysaccharide comprises polyalditol. Biodegradable non-reducing polysaccharides can very useful for formulating matrices that release the bioactive agent over a prolonged period of time.

The present invention also demonstrates the preparation of occlusions at a target site within the body that include natural biodegradable polysaccharides. These products display excellent physical characteristics and are suitable for use in applications wherein a particular function, such as bioactive agent delivery or a sealant function is desired. For example, occlusions can be prepared having viscoelastic properties. In one aspect of the invention, the occlusion has an elastic modulus value in the range of 27 kPa to 30 kPa.

In some embodiments of the invention, the methods of preparing the compositions for fabrication of matrices do not require the use of organic solvents. The use of organic solvents can be physically hazardous. Use of organic solvents can potentially destroy the activity of a bioactive agent that can be optionally included in the natural biodegradable polysaccharide-based composition.

Many of the advantageous features of the present natural biodegradable polysaccharide-containing occlusions are thought to be provided by the starting materials, in particular the natural biodegradable polysaccharides having pendent polymerisable groups, such as ethylenically unsaturated groups. In a preferred aspect, the degradable polymerizable polymers (macromers) are formed by reacting a natural biodegradable polysaccharide with a compound comprising an ethylenically unsaturated group. For example, in some cases, a natural biodegradable polysaccharide is reacted with a compound including an ethylenically unsaturated group and an isocyanate group. In another example of synthesis, a natural biodegradable polysaccharide is treated with an oxidizing agent to form a reactive aldehyde species on the polysaccharide and then reacted with a compound comprising an ethylenically unsaturated group and an amine group. Polysaccharide macromers were shown to have excellent matrix forming capabilities.

Synthesis can be carried out to provide the natural biodegradable polysaccharide with a desired quantity of pendent coupling groups. It has been found that use of a natural biodegradable polysaccharide having a predetermined amount of pendent polymerisable groups allows for the formation of an occlusion having desirable physical characteristics. Therefore, in some aspects, the invention provides natural biodegradable polysaccharides having an amount of pendent polymerisable groups of about 0.7 µmoles of polymerisable group per milligram of natural biodegradable polysaccharide. Preferably the amount of pendent polymerisable group per natural biodegradable polysaccharide is in the range of about 0.3 µmoles/mg to about 0.7 µmoles/mg. For example, amylose or maltodextrin can be subject to a synthesis reaction with a compound having an ethylenically unsaturated group to provide an amylose or maltodextrin macromer having a ethylenically unsaturated group load level in the range of about 0.3 µmoles/mg to about 0.7 µmoles/mg.

An initiator is used to promote the formation of the natural biodegradable polysaccharide matrix for occlusion formation. The initiator can be an independent compound or a pendent chemical group used to activate the coupling group pendent from the natural biodegradable polymer and promote coupling of a plurality of natural biodegradable polymers. The coupling group pendent from the natural biodegradable polysaccharide is a polymerizable group, and the initiator is used in a free radical polymerization reaction to promote crosslinking of the natural biodegradable polysaccharides together in the composition.

The initiator includes an oxidizing agent/reducing agent pair, a "redox pair," to drive polymerization of the biodegradable polysaccharide. In preparing the Biodegradable occlusion the oxidizing agent and reducing agent are combined in the presence of the biodegradable polysaccharide. One benefit of using a redox pair is that, when combined, the oxidizing agent and reducing agent can provide a particularly robust initiation system. This is advantageous as it can promote the formation of a matrix, for example, useful for article preparation, from biodegradable polysaccharide compositions having a relatively low viscosity. This can be particularly useful in many applications, especially when the biodegradable polysaccharide composition is used for the formation of an *in situ* polymerized occlusion. For example, a low viscosity composition can be passed through a small gauge delivery conduit with relative ease to provide the composition that can polymerize *in situ.*

In some aspects of the invention, the viscosity of the composition is above about 5 centi Poise (cP), or about 10 cP or greater. In other aspects of the invention the viscosity of the composition is between about 5 cP or 10 cP and about 700 cP, and in some aspects between about 5 cP or 10 cP and about 250 cP, and in some aspects between about 5 cP or 10 cP and about 45 cP. In some aspects the viscosity of the composition is above about 5 cP or 10 cP and the biodegradable polysaccharides in the composition have an average molecular weight of 500,000 Da or less, 250,000 Da or less, 100,000 Da or less, or 50,000 Da or less.

In addition, the present invention shows that redox components that can be used to form degradable matrices *in situ* are biocompatible, as demonstrated by cell viability studies.

A method for forming an occlusion can include the steps of (a) providing a first composition that includes a natural biodegradable polysaccharide comprising a coupling group and a first member of a redox pair (for example, the oxidizing agent) and then (b) mixing the first composition with second composition that includes a second member of the redox pair (for example, the reducing agent). In some aspects the second composition includes a natural biodegradable polysaccharide. For example, the first composition can include (a) a natural biodegradable polysaccharide having a coupling group and an oxidizing agent and the second composition can include a (b) natural biodegradable polysaccharide having a coupling group and a reducing agent. In some aspects, when the first composition is combined with the second composition, the final composition can be about 5 cP or greater.

The composition according to the invention is suitable for use in a method for forming a biodegradable occlusion at a target site within a body. In some cases the target site is associated with the vasculature, such as an aneurysm. The method includes the steps of (a) providing a composition comprising a natural biodegradable polysaccharide comprising a polymerizable group and a first member of a redox pair; (b) delivering the first composition at the target site within the body; and (c) contacting the composition with a second member of the redox pair. In the step of contacting, the redox pair initiates polymerization of the natural biodegradable polysaccharide to form the biodegradable occlusion at the target site.

In some aspects, the step of contacting includes delivering a second composition that includes the second member of the redox pair. Mixing of the first and second compositions at the target site results in a redox reaction and crosslinking of the natural biodegradable polysaccharides via the polymerizable groups, thereby forming the biodegradable occlusion.

In some aspects, in the step of contacting, an article configured to be delivered to the target site is associated with the second member of the redox pair. In some aspects the article is selected from the group consisting of a coil, wire, and string. In some aspects, such as for the treatment of an aneurysm target site, the article can be selected from an article that is placed within or near the aneurysm. The second member can be an oxidizing agent that can be releasable or non-releasable from the article. In the step of contacting, polymerization of the natural biodegradable polysaccharide forms a biodegradable occlusion occurs in association with the article that is inserted into the aneurysm. Formation of a biodegradable occlusion in association with, for example, a neuroaneurym coil, represents a distinct improvement over treatment with a coil alone, as the aneurysm can be substantially occluded with the formed matrix. The polymerizable compositions can be used with conventional neuroaneurym coils, but also with articles that are biodegradable.

In some aspects, the step delivering the first composition to the target site (such as a neuroaneurysm) is performed using a microcatheter having a diameter of less than 2.3 french. The inventive natural biodegradable polysaccharides of the invention allow for the preparation of very low viscosity compositions that can be passed through these small diameter microcatheters and yet polymerized to form a biodegradable occlusion with desirable physical properties.

In other aspects, the first and second members of the redox pair are combined before the composition is delivered to the target site. The present invention also shows that a matrix with desirable physical properties can be formed a significant time after the first and second members of the redox pair are combined in the presence of the natural biodegradable polysaccharides. This ample set up time is advantageous as delivery of the composition to the target site can be carried out without risk that the composition will polymerize and clog the delivery vehicle. This method includes the steps of (a) providing a composition comprising a natural biodegradable polysaccharide comprising a polymerizable group, a first member of a redox pair, and second member of a redox pair; (b) delivering the composition at the target site within the body; and (c) allowing a biodegradable occlusion to form at a target site within a body. The present invention provides compositions that can form a matrix with the properties of a semi-firm or soft gel within a time period in the range of about 20 seconds to about 10 minutes after combining the members of the redox pair.

In some aspects, the polymerizable compositions can also include a pro-fibrotic agent. Biodegradable occlusions that include a pro-fibrotic agent can promote a rapid and localized fibrotic response in the vicinity of the occlusion. This leads to the accumulation of clotting factors and formation of a fibrin clot in association with the occlusion. In turn, this improves the likelihood that the aneurysm will heal. In some aspects the pro-fibrotic agent is a polymer. The polymer can be based on a natural polymer, such as collagen, or a synthetic polymer.

Use of the natural biodegradable polysaccharides of the invention offers many advantages for occluding a desired location of the body. An occlusion with a desired degree of biodegradability can be formed *in situ* by controlling the extent of crosslinking between the polysaccharides. This allows one to control *in vivo* lifespan of the occlusion. This can also promote a healing response. In addition, the occlusion degrades by surface erosion, as opposed to bulk erosion which is common to other biodegradable polymers. In turn, this improves safety by eliminating the possibility of degraded particulates of the occlusion embolizing from the site of occlusion formation to a different location in the body. Furthermore, any unpolymerized material lost from the target site during the *in situ* process are broken down into innocuous products at a secondary location.

The oxidizing agent can be selected from inorganic or organic oxidizing agents, including enzymes; the reducing agent can be selected from inorganic or organic reducing agents, including enzymes. Exemplary oxidizing agents include peroxides, including hydrogen peroxide, metal oxides, and oxidases, such as glucose oxidase. Exemplary reducing agents include salts and derivatives of electropositive elemental metals such as Li, Na, Mg, Fe, Zn, Al, and reductases. In one aspect, the reducing agent is present in the composition at a concentration of 2.5 mM or greater when mixed with the oxidizing agent. Other reagents, such as metal or ammonium salts of persulfate, can be present in the composition to promote polymerization of the biodegradable polysaccharide.

A biodegradable occlusion, formed using redox polymerization can therefore comprise a plurality of natural biodegadable polysaccharides associated via polymerized goups, a reduced oxidizing agent, and an oxidized reducing agent.

An occlusion can be formed by an alternative method that includes combining (a) a natural biodegradable polysaccharide comprising a first coupling group with (b) a natural biodegradable polysaccharide comprising a second coupling group that is reactive with the first coupling goup, and (c) a bioactive agent. The occlusion can be partially or fully formed when reagent (a) reacts with (b) to link the natural biodegradable polysaccharides together to form the occlusion, which includes reagent (c), the bioactive agent.

In some aspects, the present invention employs the use of biodegradable microparticles that include a bioactive agent and a natural biodegradable polysaccharide, such as amylose and maltodextrin that have pendent coupling groups.

Microparticles can also be included in occlusions formed from the natural biodegadable polysaccharide. For example, microparticles can be included in an implantable medical occlusion formed in situ from the natural biodegradable polysaccharides of the invention.

Another particular advantage of the invention is that release of glucose reduces the likelihood that the process of natural biodegradable polysaccharide degradation and tissue infiltration will promote a strong inflammatory response. This is because the natural biodegradable polysaccharide-based sealant coating can degrade into materials that are non-antigenic or that have low antigenicity. Another advantage is that the degradation products are free of other materials that may cause disease, such as microbial, viral, or prionic materials potentially present in animal-derived preparations (such as bovine collagen preparations).

### Detailed Description

In one aspect, the invention provides compositions for forming a biodegradable occlusion at a target site within the body. The biodegradable occlusion can also be used for the release of bioactive agents, and in this manner can function as bioactive agent-releasing implants or depots. In some aspects, the biodegradable occlusions of the invention biodegrade within a period that is acceptable for the desired application.

The composition includes a natural biodegradable polysaccharide having a pendent polymerisable group and a first member of a redox pair. Exemplary natural biodegradable polysaccharides include amylose and maltodextrin.

As referred to herein, a "natural biodegradable polysaccharide" refers to a non-synthetic polysaccharide that is capable of being enzymatically degraded but that is generally non-enzymatically hydrolytically stable. Natural biodegradable polysaccharides include polysaccharide and/or polysaccharide derivatives that are obtained from natural sources, such as plants or animals. Natural biodegradable polysaccharides include any polysaccharide that has been processed or modified from a natural biodegradable polysaccharide (for example, maltodextrin is a natural biodegradable polysaccharide that is processed from starch). Exemplary natural biodegradable polysaccharides include amylose, maltodextrin, cyclodextrin, polyalditol, hyaluronic acid, starch, dextran, heparin, chondroitin sulfate, dermatan sulfate, heparan sulfate, keratan sulfate, dextran sulfate, pentosan polysulfate, and chitosan. Preferred polysaccharides are low molecular weight polymers that have little or no branching, such as those that are derived from and/or found in starch preparations, for example, amylose and maltodextrin. Therefore, the natural biodegradable polysaccharide can be a substantially non-branched or non-branched poly(glucopyranose) polymer.

Because of the particular utility of the amylose and maltodextrin polymers, it is preferred that natural biodegradable polysaccharides having an average molecular weight of 500,000 Da or less, 250,000 Da or less, 100,000 Da or less, or 50,000 Da or less. It is also preferred that the natural biodegradable polysaccharides have an average molecular weight of 500 Da or greater. A particularly preferred size range for the natural biodegradable polysaccharides is in the range of about 1000 Da to about 10,000 Da. Natural biodegradable polysaccharides of particular molecular weights can be obtained commercially or can be prepared. The decision of using natural biodegradable polysaccharides of a particular size range may depend on factors such as the physical characteristics of the composition (e.g., viscosity), the desired rate of degradation of the matrix, the presence of other optional moieties in the composition, for example, bioactive agents, etc.

As used herein, "amylose" or "amylose polymer" refers to a linear polymer having repeating glucopyranose units that are joined by α-1,4 linkages. Some amylose polymers can have a very small amount of branching via α-1,6 linkages (about less than 0.5% of the linkages) but still demonstrate the same physical properties as linear (unbranched) amylose polymers do. Generally amylose polymers derived from plant sources have molecular weights of about 1 X 10⁶ Da or less. Amylopectin, comparatively, is a branched polymer having repeating glucopyranose units that are joined by α-1,4 linkages to form linear portions and the linear portions are linked together via α-1,6 linkages. The branch point linkages are generally greater than 1% of the total linkages and typically 4% - 5% of the total linkages. Generally amylopectin derived from plant sources have molecular weights of 1X 10⁷ Da or greater.

Amylose can be obtained from, or is present in, a variety of sources. Typically, amylose is obtained from non-animal sources, such as plant sources. In some aspects, a purified preparation of amylose is used as starting material for the preparation of the amylose polymer having coupling groups. In other aspects, as starting material, amylose can be used in a mixture that includes other polysaccharides.

For example, in some aspects, starch preparations having a high amylose content, purified amylose, synthetically prepared amylose, or enriched amylose preparations can be used in the preparation of amylose having the coupling groups. In starch sources, amylose is typically present along with amylopectin, which is a branched polysaccharide. According to the invention, it is preferred to use compositions that include amylose, wherein the amylose is present in the composition in an amount greater than amylopectin, if present in the composition. For example, in some aspects, starch preparations having high amylose content, purified amylose, synthetically prepared amylose, or enriched amylose preparations can be used in the preparation of amylose polymer having the coupling groups. In some embodiments the composition includes a mixture of polysaccharides including amylose wherein the amylose content in the mixture of polysaccharides is 50% or greater, 60% or greater, 70% or greater, 80% or greater, or 85% or greater by weight. In other embodiments the composition includes a mixture of polysaccharides including amylose and amylopectin and wherein the amylopectin content in the mixture of polysaccharides is 30% or less, or 15% or less.

In some cases it may be desirable to use non-retrograding starches, such as waxy starch, in the current invention. The amount of amylopectin present in a starch may also be reduced by treating the starch with amylopectinase, which cleaves α-1,6 linkages resulting in the debranching of amylopectin into amylose.

In some cases a synthesis reaction can be carried out to prepare an amylose polymer having pendent coupling groups (for example, amylose with pendent ethylenically unsaturated groups) and steps may be performed before, during, and/or after the synthesis to enrich the amount of amylose, or purify the amylose.

Amylose of a particular size, or a combination of particular sizes can be used. The choice of amylose in a particular size range may depend on the application. In some embodiments amylose having an average molecular weight of 500,000 Da or less, 250,000 Da or less, 100,000 Da or less, 50,000 Da or less, preferably greater than 500 Da, or preferably in the range of about 1000 Da to about 10,000 Da is used. Amylose of particular molecular weights can be obtained commercially or can be prepared. For example, synthetic amyloses with average molecular masses of 70, 110, and 320 can be obtained from Nakano Vinegar Co., Ltd. (Aichi, Japan). The decision of using amylose of a particular size range may depend on factors such as the physical characteristics of the composition (e.g., viscosity), the desired rate of degradation of the matrix, the presence of other optional moieties in the composition (for example, bioactive agents, etc.), etc.

Maltodextrin is typically generated by hydrolyzing a starch slurry with heat-stable α-amylase at temperatures at 85 - 90 °C until the desired degree of hydrolysis is reached and then inactivating the α-amylase by a second heat treatment. The maltodextrin can be purified by filtration and then spray dried to a final product. Maltodextrins are typically characterized by their dextrose equivalent (DE) value, which is related to the degree of hydrolysis defined as: DE = MW dextrose/number-averaged MW starch hydrolysate x 100.

A starch preparation that has been totally hydrolyzed to dextrose (glucose) has a DE of 100, where as starch has a DE of about zero. A DE of greater than 0 but less than 100 characterizes the mean-average molecular weight of a starch hydrolysate, and maltodextrins are considered to have a DE of less than 20. Maltodextrins of various molecular weights, for example, in the range of about 500 - 5000 Da are commercially available (for example, from CarboMer, San Diego, CA).

Another contemplated class of natural biodegradable polysaccharides is natural biodegradable non-reducing polysaccharides. A non-reducing polysaccharide can provide an inert matrix thereby improving the stability of sensitive bioactive agents, such as proteins and enzymes. A non-reducing polysaccharide refers to a polymer of non-reducing disaccharides (two monosaccharides linked through their anomeric centers) such as trehalose (α-D-glucopyranosyl α-D-glucopyranoside) and sucrose (β-D-ftuctofuranosyl α-D-glucopyranoside). An exemplary non-reducing polysaccharide comprises polyalditol which is available from GPC (Muscatine, Iowa). In another aspect, the polysaccharide is a glucopyranosyl polymer, such as a polymer that includes repeating (1→3)O-β-D-glucopyranosyl units.

In some aspects, the compositions can include natural biodegradable polysaccharides that include chemical modifications other than the pendent coupling group. To exemplify this aspect, modified amylose having esterified hydroxyl groups can be prepared and used in compositions in association with the methods of the invention. Other natural biodegradable polysaccharides having hydroxyl groups may be modified in the same manner. These types of modifications can change or improve the properties of the natural biodegradable polysaccharide making for a composition that is particularly suitable for a desired application. Many chemically modified amylose polymers, such as chemically modified starch, have at least been considered acceptable food additives.

As used herein, "modified natural biodegradable polysaccharides" refers to chemical modifications to the natural biodegradable polysaccharide that are different than those provided by the coupling group or the initiator group. Modified amylose polymers having a coupling group (and/or initiator group) can be used in the compositions and methods of the invention.

To exemplify this aspect, modified amylose is described. By chemically modifying the hydroxyl groups of the amylose, the physical properties of the amylose can be altered. The hydroxyl groups of amylose allow for extensive hydrogen bonding between amylose polymers in solution and can result in viscous solutions that are observed upon heating and then cooling amylose-containing compositions such as starch in solution (retrograding). The hydroxyl groups of amylose can be modified to reduce or eliminate hydrogen bonding between molecules thereby changing the physical properties of amylose in solution.

Therefore, in some embodiments the natural biodegradable polysaccharides, such as amylose, can include one or more modifications to the hydroxyl groups wherein the modifications are different than those provided by coupling group. Modifications include esterification with acetic anhydride (and adipic acid), succinic anhydride, 1-octenylsuccinic anhydride, phosphoryl chloride, sodium trimetaphosphate, sodium tripolyphosphate, and sodium monophosphate; etherification with propylene oxide, acid modification with hydrochloric acid and sulfuric acids; and bleaching or oxidation with hydrogen peroxide, peracetic acid, potassium permanganate, and sodium hypochlorite.

Examples of modified amylose polymers include carboxymethyl amylose, carboxyethyl amylose, ethyl amylose, methyl amylose, hydroxyethyl amylose, hydroxypropyl amylose, acetyl amylose, amino alkyl amylose, allyl amylose, and oxidized amylose. Other modified amylose polymers include succinate amylose and oxtenyl succinate amylose.

In another aspect of the invention, the natural biodegradable polysaccharide is modified with a hydrophobic moiety in order to provide a biodegradable matrix having hydrophobic properties. Exemplary hydrophobic moieties include those previously listed, fatty acids and derivatives thereof, and C₂-C₁₈ alkyl chains. A polysaccharide, such as amylose or maltodextrin, can be modified with a compound having a hydrophobic moiety, such as a fatty acid anhydride. The hydroxyl group of a polysaccharide can also cause the ring opening of lactones to provide pendent open-chain hydroxy esters.

In some aspects, the hydrophobic moiety pendent from the natural biodegradable has properties of a bioactive agent. The hydrophobic moiety can be hydrolyzed from the natural biodegradable polymer and released from the matrix to provide a therapeutic effect. One example of a therapeutically useful hydrophobic moiety is butyric acid, which has been shown to elicit tumor cell differentiation and apoptosis, and is thought to be useful for the treatment of cancer and other blood diseases. The hydrophobic moiety that provides a therapeutic effect can also be a natural compound (such as butyric acid). Therefore, degradation of the matrix having a coupled therapeutic agent can result in all natural degradation products.

According to the invention; a natural biodegradable polysaccharide that includes a pendent polymerisable group is used to form an occlusion. Other polysaccharides can also be present in the composition. For example, the two or more natural biodegradable polysaccharides are used to form an occlusion. Examples include amylose and one or more other natural biodegradable polysaccharide(s), and maltodextrin and one or more other natural biodegradable polysaccharide(s); in one aspect the composition includes a mixture of amylose and maltodextrin, optionally with another natural biodegradable polysaccharide.

In one preferred embodiment, amylose or maltodextrin is the primary polysaccharide. In some embodiments, the composition includes a mixture of polysaccharides including amylose or maltodextrin and the amylose or maltodextrin content in the mixture of polysaccharides is 50% or greater, 60% or greater, 70% or greater, 80% or greater, or 85% or greater by weight.

Purified or enriched amylose preparations can be obtained commercially or can be prepared using standard biochemical techniques such as chromatography. In some aspects, high-amylose cornstarch can be used.

As used herein, "coupling group" can include (1) a chemical group that is able to form a reactive species that can react with the same or similar chemical group to form a bond that is able to couple the natural biodegradable polysaccharides together (for example, wherein the formation of a reactive species can be promoted by an initiator); or (2) a pair of two different chemical groups that are able to specifically react to form a bond that is able to couple the natural biodegradable polysaccharides together. The coupling group can be attached to any suitable natural biodegradable polysaccharide, including the amylose and maltodextrin polymers as exemplified herein.

The natural biodegradable polysaccharides of the invention include at least one, and more typically more than one, pendent polymerisable group per natural biodegradable polysaccharide, allowing for a plurality of natural biodegradable polysaccharides to be coupled in linear and/or branched manner. In some preferred embodiments, the natural biodegradable polysaccharide includes two or more pendent polymerisable groups.

The coupling group on the natural biodegradable polysaccharide is a polymerizable group. In a free radical polymerization reaction the polymerizable group can couple natural biodegradable polysaccharides together in the composition, thereby forming a biodegradable natural biodegradable polysaccharide matrix.

A preferred polymerizable group is an ethylenically unsaturated group. Suitable ethylenically unsaturated groups include vinyl groups, acrylate groups, methacrylate groups, ethacrylate groups, 2-phenyl acrylate groups, acrylamide groups, methacrylamide groups, itaconate groups, and styrene groups. Combinations of different ethylenically unsaturated groups can be present on a natural biodegradable polysaccharide, such as amylose or maltodextrin.

In preparing the natural Biodegradable polysaccharide, having pendent polymerisable groups any suitable synthesis procedure can be used. Suitable synthetic schemes typically involve reaction of, for example, hydroxyl groups on the natural biodegradable polysaccharide, such as amylose or maltodextrin. Synthetic procedures can be modified to produce a desired number of polymerisable groups pendent from the natural biodegradable polysaccharide backbone. For example, the hydroxyl groups can be reacted with a polymerisable group-containing compound or can be modified to be reactive with a coupling group-containing compound. The number and/or density of acrylate groups can be controlled using the present method, for example, by controlling the relative concentration of reactive moiety to saccharide group content.

In some modes of practice, the biodegradable polysaccharides have an amount of pendent polymerisable groups of about 0.7 µmoles of coupling group per milligram of natural biodegradable polysaccharide. In a preferred aspect, the amount of polymerisable group per natural biodegradable polysaccharide is in the range of about 0.3 µmoles/mg to about 0.7 µmoles/mg. For example, amylose or maltodextrin can be reacted with an acrylate groups-containing compound to provide an amylose or maltodextrin macromer having a acrylate group load level in the range of about 0.3 µmoles/mg to about 0.7 µmoles/mg.

As used herein, an "initiator" refers to a compound, or more than one compound, that is capable of promoting the formation of a reactive species from the coupling group. For example, the initiator can promote a free radical reaction of natural biodegradable polysaccharide having a coupling group. In a comparative example the initiator is a photoreactive group (photoinitiator) that is activated by radiation. In some embodiments, the initiator can be an "initiator polymer" that includes a polymer having a backbone and one or more initiator groups pendent from the backbone of the polymer.

In some embodiments, photoinitiation occurs using groups that promote an intra- or intermolecular hydrogen abstraction reaction. This initiation system can be used without additional energy transfer acceptor molecules and utilizing nonspecific hydrogen abstraction, but is more commonly used with an energy transfer acceptor, typically a tertiary amine, which results in the formation of both aminoalkyl radicals and ketyl radicals. Examples of molecules exhibiting hydrogen abstraction reactivity and useful in a polymeric initiating system, include analogs of benzophenone, thioxanthone, and camphorquinone.

A photoinitiator can include one or more charged groups. The presence of charged groups can increase the solubility of the photoinitiator (which can contain photoreactive groups such as aryl ketones) in an aqueous system and therefore provide for an improved composition. Suitable charged groups include, for example, salts of organic acids, such as sulfonate, phosphonate, carboxylate, and the like, and onium groups, such as quaternary ammonium, sulfonium, phosphonium, protonated amine, and the like. According to this embodiment, a suitable photoinitiator can include, for example, one or more aryl ketone photogroups selected from acetophenone, benzophenone, anthraquinone, anthrone, anthrone-like heterocycles, and derivatives thereof; and one or more charged groups, for example, as described herein. Examples of these types of water-soluble photoinitiators have been described in U.S. Patent No. 6,077,698.

A photoinitiator can be a compound that is activated by long-wavelength ultraviolet (UV) and visible light wavelengths. For example, the initiator includes a photoreducible or photo-oxidizable dye. Photoreducible dyes can also be used in conjunction with a compound such as a tertiary amine. The tertiary amine intercepts the induced triplet producing the radical anion of the dye and the radical cation of the tertiary amine. Examples of molecules exhibiting photosensitization reactivity and useful as an initiator include acridine orange, camphorquinone, ethyl eosin, eosin Y, erythrosine, fluorescein, methylene green, methylene blue, phloxime, riboflavin, rose bengal, thionine, and xanthine dyes. Use of these types of photoinitiators can be particularly advantageous when a light-sensitive bioactive agent is included in the composition.

Redox initiators are used to promote the polymerization of the natural biodegradable polymers having pendent polymerisable groups. In general, combinations of organic and inorganic oxidizers, and organic and inorganic reducing agents are used to generate radicals for polymerization. A description of redox initiation can be found in Principles of Polymerization, 2nd Edition, Odian G., John Wiley and Sons, pgs 201-204, (1981).

In some cases, the initiator can be included In a base coating and the natural biodegradable polysaccharide or composition that includes the natural biodegradable polysaccharide can be disposed on the base coating. For example, a coated layer that includes the natural biodegradable polysaccharide can be formed on a coated layer that includes a synthetic polymer. The synthetic polymer can be a hydrophilic polymer such as poly(vinylpyrrolidone), poly(acrylamide), or copolymers thereof. In some aspects tho synthetic polymer is formed using photoreactive groups, such as photoreactive groups that are pendent from the synthetic polymer, which can be used to covalently bond the synthetic polymer to a surface of the article.

In some aspects the polymerization initiator is a polymer that includes an initiator group (herein referred to as an "initiator polymer"). The polymeric portion of the initiator polymer can be obtained or prepared to have particular properties or features that are desirable for use with a composition, such as a sealant composition. For example, the polymeric portion of the initiator polymer can have hydrophilic or amphoteric properties, it can include pendent charged groups, or it can have groups that allow it to interact with a particular surface. Optionally, or additionally, the polymer can change or improve the properties of the matrix that is formed by the amylose polymer having coupling groups. For example, the initiator polymer can change the elasticity, flexibility, wettability, or softness (or combinations thereof) of the matrix. Certain polymers, as described herein, are useful as plasticizing agents for matrices that include natural biodegradable polysaccharides. Initiator groups can be added to these plasticizing polymers and used in the compositions and methods of the invention.

For example, in some aspects an initiator can be pendent from a natural biodegradable polysaccharide. Therefore, the natural biodegradable polysaccharide is able to promote activation of polymerizable groups that are pendent from other natural biodegradable polysaccharides and promote the formation of a natural biodegradable polysaccharide matrix.

In other cases, the polymeric portion of the initiator polymer can include, for example, acrylamide and methacrylamide monomeric units, or derivatives thereof. In some embodiments, the composition includes an initiator polymer having a photoreactive group and a polymeric portion selected from the group of acrylamide and methacrylamide polymers and copolymers.

The initiator includes an oxidizing agent/reducing agent pair, a "redox pair," to drive polymerization of the biodegradable polysaccharide. In this case, polymerization of the biodegradable polysaccharide is carried out upon combining one or more oxidizing agents with one or more reducing agents. Other compounds can be included in the composition to promote polymerization of the biodegradable polysaccharides.

When combined, the oxidizing agent and reducing agent can provide a particularly robust initiation system and can drive the formation of a polymerized matrix of polysaccharides from a composition having a low viscosity. A polysaccharide composition with a low viscosity may be due to a low concentration of polysaccharide in the composition, a polysaccharide having a low average molecular weight, or combinations thereof. Matrix formation from a polysaccharide composition having a low viscosity is particularly advantageous in many applications, especially for *in situ* polymerization. In some aspects of the invention, a low viscosity polysaccharide composition is passed through a small gauge delivery conduit, such as a needle or a catheter, wherein the redox pair causes the polymerization of the polysaccharides *in situ.*

In some aspects of the invention, the viscosity of the composition is above about 5 cP, or about 10 cP or greater. In other aspects of the invention the viscosity of the composition is between about 5 cP or 10 cP and about 700 cP, or between about 5 cP or 10 cP and about 250 cP, or between about 5 cP or 10 cP and about 45 cP.

In some modes of practice, in order to promote polymerization of the biodegradable polysaccharides in a composition to form a matrix, the oxidizing agent is added to the reducing agent in the presence of the one or more biodegradable polysaccharides. For example, a composition including a biodegradable polysaccharide and a reducing agent is added to a composition including an oxidizing agent, or a composition including a biodegradable polysaccharide and an oxidizing agent is added to a composition containing a reducing agent. One desirable method of preparing a matrix is to combine a composition including a biodegradable polysaccharide and an oxidizing agent with a composition including a biodegradable polysaccharide and a reducing agent For purposes of describing this method, the terms "first composition" and "second composition" can be used.

The oxidizing agent can be selected from inorganic or organic oxidizing agents, including enzymes; the reducing agent can be selected from inorganic or organic reducing agents, including enzymes. Exemplary oxidizing agents include peroxides, including hydrogen peroxide and di-tert-butyl peroxide, metal oxides, and oxidases, including glucose oxidase.

Exemplary reducing agents and co-reducing agents include salts and derivatives of electropositive elemental metals such as Li, Na, Mg, Fe, Zn, Al, including ferrous salts such as ferrous lactate, ferrous gluconate, and ferrous acetate, organic acids and derivatives thereof such as ascorbic acid, folic acid, and pantothenic acid, reductases, and amine compounds. Other reducing agents of co-reductants include erythrobate and α-tocopherol.

In some aspects the redox pair includes an oxidase:reductant combination. Exemplary oxidase:reductant combinations include: (a) glycollate oxidase:glycollate/L-lactate/D-lactate/(+)-mandalate; (b) lactate oxidase:L-lactate; (c) glucose oxidase:beta.-D-glucose/2-dioxy-D-glucose/6-methyl-D-glucose; (d) hexose oxidase:β-D-glucose/D-galactose/D-mannose; (e) galactose oxidase:D-galactose/lactose; (f) L-2-hydroxyacid oxidase:L-2-hydroxyacid; (g) aldehyde oxidase:formaldehyde/acetaldehyde; (h) xanthine oxidase:purine/hypoxanthine/xanthine; (i) pyruvate oxidase:pyruvate; (j) oxalate oxidase :oxalate; (k) dihydro-orotate-dehydrogenase:L-4, 5-dihydro-orotate/NAD; (1) D-aspartate oxidase:D-aspartate/D-glutamate; (m) L-Amino-acid oxidase:L-methionine/L-phenylalanine/2-hydroxy acids/L-lactate; (n) D-Amino acid oxidase:D-alanine/-valine/D-proline; (o) monoamine oxidase:monoamine/benzlamine/octylamine; (p) diamine oxidase:diamines/spermidine/tyramine; (q) alcohol oxidase:ethanol/methanol (r) carbohydrate oxidase:D-glucose/D-glucopyranose/D-xylopyranose/I-sorbose/alpha.-D-gluconolactone (s) NADH oxidase:NADH; (t) malate oxidase:L-malate; (u) cholesterol oxidase:cholesterol; (v) thiol oxidase:thiol; and (w) ascorbate oxidase:L-ascorbate.

In one mode of practice, the reducing agent is present at a concentration of about 2.5 mM or greater when the reducing agent is mixed with the oxidizing agent. Prior to mixing, the reducing agent can be present in a composition at a concentration of, for example, 5 mM or greater.

Other reagents can be present in the composition to promote polymerization of the biodegradable polysaccharide. Other polymerization promoting compounds can be included in the composition, such as metal or ammonium salts of persulfate.

Optionally, the compositions and methods of the invention can include polymerization accelerants that can improve the efficiency of polymerization. Examples of useful accelerants include N-vinyl compounds, particularly N-vinyl pyrrolidone and N-vinyl caprolactam. Such accelerants can be used, for instance, at a concentration of between about 0.01% and about 5%, and preferably between about 0.05% and about 0.5%, by weight, based on the volume of the composition.

The viscosities of biodegradable polysaccharide in the first and second compositions can be the same or can be different. Generally, though, it has been observed that good mixing and subsequent matrix formation is obtained when the compositions have the same or similar viscosities. In this regard, if the same biodegradable polymer is used in the first and second compositions, the concentration of the biodegradable polymer may be the same or different.

In some methods of use, polymerization of the composition is promoted *in situ,* such as at a target site for forming a biodegradable occlusion with the polymerized mass of material. To illustrate this aspect, the method can be performed for the treatment of an aneurysm target site. Filling of an aneurysm with the biodegradable materials of the invention can at least stabilize the aneurysm and therefore reduce the likelihood that the aneurysm will rupture of further increase in size.

In the process, first and second compositions are delivered to the aneurysm target site via microcatheters. Microcatheters generally have very small diameters, such as about 5 french (fr) or less. ("French size" generally refers to units of outer diameter of a catheter; Fr size X 0.33 = outer diameter of the catheter in mm.) In some aspects, the neuroaneurysm target site and the vasculature through which the catheters are navigated, dictates that very small microcatheters be used, for example having a size of about 2.3 french or less, such as in the range of about 1.7 french to about 2.3 french (commercially available from, for example, Boston Scientific Excelsior SL-10 #168189). The compositions of the present invention, which can be used at low viscosities to form biodegradable occlusions, can be delivered though microcatheters of these sizes at an acceptable flow rate without the risk of clogging the lumen of the catheters.

In practice, a dual lumen microcatheter can be inserted into the vasculature of a subject and navigated to place the distal end of the microcatheter at the neuroaneurysm target site. First and second compositions that include natural biodegradable polysaccharides and, individually, an oxidizing agent, and a reducing agent can be delivered to and mixed within the aneurysm. Based on the polymerizable compositions of the inventions, it has been found that these compositions can be delivered through very small catheters. For example, the composition can be delivered through a 1.7 fr catheter. (The inner diameter of a 1.7 fr catheter is 0.42 mm and the outer diameter is 0.56 mm.) Furthermore, the composition can be delivered at very good flow rates. For example, the flow rate can be up about 40 uL/sec to about 50 uL/sec. Given this, use of the inventive compositions can allow for the treatment of aneurysms accessible via smaller vasculature in a very efficient manner.

In another mode of practice, the first and second members of the redox pair are combined before the composition is delivered to the target site. Compositions are prepared that allow for mixing and delivery of the composition to the target site before the composition polymerizes into a matrix. In these aspects a preferred redox pair includes an oxidant selected from a metal, potassium, or ammonium salt of persulfate and an amine compound, such as N,N,N',N'-Tetramethylethylenediamine (TEMED). The oxidant is desirably present in the composition at a concentration of about 5 mg/mL or greater, about 10 mg/mL or greater, about 15 mg/mL or greater, or about 30 mg/mL or greater. The amine compound, such as TEMED, is desirably present in the composition in an amount of about 20 □L/mL or greater. An exemplary amount of natural biodegradable polysaccharide, such as polyalditol acrylate, present in the composition is about 500 mg/mL or greater.

Following mixing of the member of the redox pair, a period of time elapses before the composition sets up into a matrix, which can have semi-firm or sol gel properties. The period of time can be about 20 seconds or greater, 30 seconds or greater, 45 seconds or greater, 50 seconds or greater, 60 seconds or greater, 120 seconds or greater, 240 seconds or greater, 360 seconds or greater, or up to about 600 seconds. In this period of time, the composition can be mixed and delivered to a target site in the body, such as an aneurysm. After the composition is delivered to the target site, a matrix in the form of a biodegradable occlusion is formed.

While the compositions of the present invention are particularly suitable for being delivered via a small diameter catheter, the compositions can also be delivered via larger diameter catheters. Larger diameter catheters can be used to deliver the inventive compositions to one or more portions of the urogenital system.

The amount of composition to be delivered to the aneurysm can vary and will depend on the size of the aneurysm. The delivery results in a localized redox reaction and polymerization of the composition to form a biodegradable occlusion in the aneurysm. The occlusion can seal off the aneurysm and prevent further enlargement.

As another way of promoting polymerization, a composition including the biodegradable polysaccharides and a first member of a redox pair, such as a reducing agent, can be contacted with an article that is associated with a second member of a redox pair, such as an oxidizing agent. The article can be a portion of medical device, such as those described herein, or any sort of article that can be used in a medical procedure.

In some cases, the second member of the redox pair is releasable from the article. The second member can be releasable by diffusion from the article itself, for example, if the article is impregnated with the second member. Alternatively, the second member can be releasable from a coating formed on the second member. Degradable material can also be used to form the article that includes the second member. The second member can be releasable from a biodegradable article or a biodegradable coating that is formed on an article. The article or coating can be formed from the natural biodegradable polysaccharides as described herein along with the second member.

In other cases, the second member is non-releasably bound to the article. For example, the second member may be covalently bonded to the surface of the article. When the article is placed in contact with the composition containing the natural biodegradable polysaccharide, a redox reaction can occur near the surface of the article and propagate the polymerization of the polysaccharide from the surface to form a matrix in association with the article.

In some desired modes of practice the second member is an organic oxidizing compound, such as di-tert-butyl peroxide, that is immobilized on the article.

In some aspects the composition including the natural biodegradable polysaccharide is used in conjunction with an occlusion device, in methods for promoting the occlusion of any sort of target area within the body. For example, the occlusion device can be placed at a location within the vasculature of a subject. As another example, the occlusion device can be placed at a location within one or more portions of the urogenital system of a subject (such as the fallopian tube of a female subject). The composition may be used to improve the function of the occlusion device at the target site. For example, a biodegradable matrix may be formed in association with the occlusion device at a target site.

The occlusion device may serve as a way to facilitate polymerization of the polysaccharide composition. For example, a member of a redox pair can be associated with one or more portions of the occlusion device. The member may be releasable or non-releasable from the occlusion device.

The occlusion device, or a portion thereof, can be configured to be placed within the vasculature (a implantable vascular device), such as in an artery, vein, fistula, or aneurysm. In some cases the occlusion device is selected from vascular occlusion coils, wires, or strings that can be inserted into aneurysms. Some specific vascular occlusion devices include detachable embolization coils. In some cases the device is a stent.

Alternatively, the device, or a portion thereof, can be configured to be placed within other body lumens, such as the fallopian tubes, bile ducts, etc. For example, the occlusion device can be placed at one or more portions of the urogenital system. Some exemplary implantable urogenital devices are used for birth control, for example, fabric-containing occlusive coils which are inserted into the fallopian tubes by hysteroscopy (Conceptus, Mountain View, CA).

Vascular occulsion devices can be in the form of wires, coils, braids, strings, and the like; some vascular occulsion devices have a helically wound configuration. Exemplary coils are generally 2.2 mm or less in diameter, more particularly in the range of 0.2 mm to 2.2 mm and can be composed of wires 1.25 mm or less in diameter, for example in the range of 0.125 mm to 1.25 mm. Lengths of vascular occulsion devices typically range from 0.5 to 100 centimeters.

Vascular occlusion devices are commonly prepared from metals such as platinum, gold, or tungsten, although other metals such as rhenium, palladium, rhodium, ruthenium, titanium, nickel, and alloys of these metals, such as stainless steel, titanium/nickel, and nitinol alloys, can be used.

The vascular occulsion device can also include a polymeric material. Particularly useful devices include polymers having hydrogel properties. Exemplary polymers include poly(urethanes), poly(acrylates), poly(methacrylates), poly(vinylpyrrolidone), cellulose acetate, ethylene vinyl alcohol copolymers, poly(acrylonitrile), poly(vinylacetate), cellulose acetate butyrate, nitrocellulose, copolymers of urethane/carbonate, copolymers of styrene/maleic acid, or mixtures thereof.

Formation of a biodegradable occlusion in association with a vascular occlusion device is illustrated by the following procedure. A neuroaneurysm occlusion device having a distal coil portion that includes an oxidizing agent is advanced to an aneurysm via the vasculature. A microcatheter is also advanced to the aneursym. The coil and microcatheter can be advanced to the aneurysm simultaneously or one may precede the other. If the oxidizing agent is releasable, prior to delivering the polymerizable composition, the coil may reside in the aneurysm for a period of time sufficient for the oxidizing agent to be released and diffuse within the aneurismal space. Compositions that include the polysaccharide and a reducing agent can then be delivered to the aneurysm via a microcatheter.

The distal portion of the coil can be separated from the proximal portion via processes similar to those used with Gugliemi Detachable Coils (GDCs). An electrostatic charge can be delivered to detach the coil portion that is inserted into the aneurysm.

In an alternative method, the biodegradable occlusion can be formed by a method that includes step of (a) delivering a first composition having a natural biodegradable polysaccharide comprising a first pendent polymerisable coupling group to the target site and (b) delivering a second composition having a natural biodegradable polysaccharide comprising a second coupling group that is reactive with the first coupling group. Mixing of the first and second compositions at the target site results in crosslinking and formation of the biodegradable occlusion. Suitable first and second coupling groups are described herein.

In some aspects, the polymerizable compositions can also include a pro-fibrotic agent. The pro-fibrotic agent can promote a rapid and localized fibrotic response in the vicinity of the formed occlusion. This can lead to the accumulation of clotting factors, such as by the adhesion of platelets, and formation of a fibrin clot in association with the occlusion. In combination with the space filling function provided by the polymerized mass of material, the formed clot may further sealing off the aneurysm. As the occlusion degrades and tissue is formed in the vicinity of the occlusion, a healing process may occur, wherein the aneurysm shrinks in size, or disappears altogether. The profibrotic agent could promote the formation of neointima at the neck of the occluded aneurysm. Gradually, this could lead to the ingrowth of tissue into the matrix, resulting in the formation of an occlusion of natural tissue. Such a healing process would be greatly beneficial to a subject. The profibrotic agent can be present in an amount sufficient to provide a desired pro-fibrotic response in the vicinity of the formed occlusion.

In some aspects of the invention, the pro-fibrotic agent is a polymer. The profibrotic polymer can be a natural polymer, such as a peptide or protein. Examples of pro-fibrotic peptides or proteins include, but are not limited to, for example, thrombin and collagen, such as, recombinant human collagen (FibroGen, South San Francisco, CA). Collagen peptides and modified collagen can be used in the preparation of the pro-fibrotic matrix. Other contemplated pro-fibrotic polypeptides are described herein.

In one embodiment the pro-fibrotic matrix includes a non-animal derived profibrotic polypeptide. As used herein, an "animal" refers to a non-human animal that typically is used as livestock and includes animals such as cows (bovine), pig (porcine), and chicken, from which collagen is typically extracted.

Other useful pro-fibrotic agents can include platelet factors 1-4, platelet activating factor (acetyl glyceryl ether phosphoryl choline); P-selectin and von Willebrand factor (vWF); tissue factor; plasminogen activator initiator-1; thromboxane; procoagulant thrombin-like enzymes including cerastotin and afaâcytin; phospholipase A2; Ca2+-dependent lectins (C-type lectin); factors that bind glycoprotein receptors and induce aggregation including aggretin, rhodocytin, aggregoserpentin, triwaglerin, and equinatoxin; glycoprotein Ib agonists including mamushigin and alboaggregin; vWF interacting factors including botrocetin, bitiscetin, cerastotin, and ecarin.

Other factors, including protein factors, that are involved in the clotting cascade include coagulation factors I - XIII (for example, fibrinogen, prothrombin, tissue thromboplastin, calcium, proaccelerin (accelerator globulin), proconvertin (serum prothrombin conversion accelerator), antihemophilic factor, plasma thromboplastin component, Stuart factor (autoprothrombin C), plasma thromboplastin antecedent (PTA), Hageman factor, and fibrin-stabilizing factor (FSF, fibrinase, protransglutaminase)).

In some aspects, the pro-fibrotic agent is a pro-fibrotic cationic polymer.
The pro-fibrotic cationic polymer is preferably a polymer conveying a positive charge sufficient to attract platelets and clotting factors. The pro-fibrotic cationic polymer can include, for example, primary amine groups. Exemplary cationic polymers include dextrans and polyimines having amine groups, for example, DEAE dextran (diethyleneaminoethyl dextran) and polyethyleneimine (PEI). A preferred synthetic pro-fibrotic cationic polymer is polyethyleneimine. Exemplary naturally-occurring-cationic polymers include chitin and chitosan (D-acetylated chitin). The pro-fibrotic cationic polymer can be a homopolymer or a copolymer. The pro-fibrotic matrix can also include blends of different cationic polymers that can promote a pro-fibrotic response.

If a pro-fibrotic polypeptide is used, a biodegradable composition can be prepared that improves the stability of the polypeptide that is in association with the polysaccharide, in unpolymerized and/or polymerized form. For example, a pro-fibrotic protein such as collagen can be included in a composition with a polyalditol macromer, which is a non-reducing polysaccharide. In some ways, stability may be improved by maintaining proper disulfide bonding in proteins having cystiene residues.

A biodegradable composition can also be prepared using pro-fibrotic macromers. For example, a pro-fibrotic polypeptide macromer can be included in the composition and polymerized along with the natural biodegradable polysaccharide. Polypeptide in macromer form can be included in the composition at concentrations greater than the polypeptide in native form. A collagen macromer can be prepared by various techniques, including those described herein.

During delivery of the composition, while efforts are made at maintaining the delivered polymeric material at the target site, it is conceivable that some leakage of unpolymerized or partially polymerized material may occur. The compositions of the invention are clearly advantageous in that any unpolymerized or partially polymerized material lost from the target site can be degraded into innocuous products elsewhere in the body.

A radiopacifying agent can also be included in a natural biodegradable polysaccharide composition. The radiopacifying agent can improve imagining of an article that is implanted, inserted, or formed within the body. For example, an imaging agent can be included in a biodegradable device that is formed using the natural biodegradable polysaccharide. This can improve detection of the device during and/or after insertion to a desired location in the body. An imaging agent can be included in a biodegradable matrix, such as an occlusion, that is formed at a target location in the body, such as an aneurysm. The imaging agent can be useful to determine the formation of the occlusion, as well as aspects of the tissue that the natural biodegradable polysaccharide is in contact with.

In some specific aspects, the radiopacifying agent comprises iodine. Polysaccharide compositions of the invention have been found to complex iodine, thereby providing a useful way of improving the imaging of an article in the body. Release of iodine during or after degradation of the polysaccharide matrix is non-toxic.

The radiopacifying agent can be iodine, or a secondary compound, such as a commercially available iodine-containing radiopacifying agent.

The radiopacifying agent can also be a radioisotope, such as I¹²⁵. The radioisotope may also serve a secondary function, such as the radiotherapeutic treatment of tissue that is in contact with the polymerized natural biodegradable polysaccharide.

In some aspects, an aqueous composition that includes the natural biodegradable polysaccharide, such as amylose or maltodextrin having pendent polymerisable groups, and a bioactive agent is obtained and used in the method of forming an occlusion. This composition can be prepared by mixing a bioactive agent, such as a water-soluble small molecule, a protein, or a nucleic acid, with the natural biodegradable polysaccharide.

According to the invention, the natural biodegradable polysaccharide that includes a pendent polymerisable group is used to form an occlusion at a target site within the body. Other polysaccharides can also be present in the composition. For example, the composition can include two different natural biodegradable polysaccharides, or more than two different natural biodegradable polysaccharides. For example, in some cases the natural biodegradable polysaccharide (such as amylose or maltodextrin) can be present in the occlusion composition along with another biodegradable polymer (i.e., a secondary polymer), or more than one other biodegradable polymer. An additional polymer or polymers can be used to alter the properties of the matrix, or serve as bulk polymers to alter the volume of the matrix. For example, other biodegradable polysaccharides can be used in combination with the amylose polymer. These include hyaluronic acid, dextran, starch, amylose (for example, non-derivitized), amylopectin, cellulose, xanthan, pullulan, chitosan, pectin, inulin, alginates, and heparin.

The concentration of the natural biodegradable polysaccharide in the composition can be chosen to provide an article having a desired density of crosslinked natural biodegradable polysaccharide. In some embodiments, the concentration of natural biodegradable polysaccharide in the composition can depend on the type or nature of the bioactive agent that is included in the composition. In some embodiments the natural biodegradable polysaccharide having the polymerisable groups is present in the composition at a concentration in the range of 5-100% (w/v), and 5-50%, and in more specific embodiments in the range of 10-20% and in other embodiments in the range of 20 - 50% (w/v).

Other polymers or non-polymeric compounds can be included in the composition that can change or improve the properties of the matrix that is formed by the natural biodegradable polysaccharide having coupling groups in order to change the elasticity, flexibility, wettability, or adherent properties, (or combinations thereof) of the matrix.

For example, in order to improve the properties of a matrix, it is possible to include in the mixture one or a combination of plasticizing agents. Suitable plasticizing agents include glycerol, diethylene glycol, sorbitol, sorbitol esters, maltitol, sucrose, fructose, invert sugars, corn syrup, and mixtures thereof. The amount and type of plasticizing agents can be readily determined using known standards and techniques.

In many aspects of the invention, the biodegradable occlusion includes one or more bioactive agents. The bioactive agent can be dispersed within biodegradable article itself. Alternatively, the bioactive agent can be present in microparticles. The bioactive agent can be delivered upon degradation of the natural biodegradable polysaccharide and/or microparticles.

The term "bioactive agent" refers to a peptide, protein, carbohydrate, nucleic acid, lipid, polysaccharide, synthetic inorganic or organic molecule, viral particle, cell, or combinations thereof, that causes a biological effect when administered *in vivo* to an animal, including but not limited to birds and mammals, including humans. Nonlimiting examples are antigens, enzymes, hormones, receptors, peptides, and gene therapy agents. Examples of suitable gene therapy agents include (a) therapeutic nucleic acids, including antisense DNA, antisense RNA, and interference RNA, and (b) nucleic acids encoding therapeutic gene products, including plasmid DNA and viral fragments, along with associated promoters and excipients. Examples of other molecules that can be incorporated include nucleosides, nucleotides, vitamins, minerals, and steroids.

Although not limited to such, the can be used for delivering bioactive agents that are large hydrophilic molecules, such as polypeptides (including proteins and peptides), nucleic acids (including DNA and RNA), polysaccharides (including heparin), as well as particles, such as viral particles, and cells. In one aspect, the bioactive agent has a molecular weight of about 10,000 or greater.

Classes of bioactive agents which can be incorporated into biodegradable matricess (both the natural biodegradable matrix and/or the biodegradable microparticles) of this invention include, but are not limited to: ACE inhibitors, actin inhibitors, analgesics, anesthetics, anti-hypertensives, anti polymerases, antisecretory agents, anti-AIDS substances, antibiotics, anti-cancer substances, anti-cholinergics, anti-coagulants, anticonvulsants, anti-depressants, anti-emetics, antifungals, anti-glaucoma solutes, antihistamines, antihypertensive agents, anti-inflammatory agents (such as NSAIDs), anti metabolites, antimitotics, antioxidizing agents, anti-parasite and/or anti-Parkinson substances, antiproliferatives (including antiangiogenesis agents), anti-protozoal solutes, anti-psychotic substances, anti-pyretics, antiseptics, anti-spasmodics, antiviral agents, calcium channel blockers, cell response modifiers, chelators, chemotherapeutic agents, dopamine agonists, extracellular matrix components, fibrinolytic agents, free radical scavengers, growth hormone antagonists, hypnotics, immunosuppressive agents, immunotoxins, inhibitors of surface glycoprotein receptors, microtubule inhibitors, miotics, muscle contractants, muscle relaxants, neurotoxins, neurotransmitters, opioids, photodynamic therapy agents, prostaglandins, remodeling inhibitors, statins, steroids, thrombolytic agents, tranquilizers, vasodilators, and vasospasm inhibitors.

Antibiotics are art recognized and are substances which inhibit the growth of or kill microorganisms. Examples of antibiotics include penicillin, tetracycline, chloramphenicol, minocycline, doxycycline, vancomycin, bacitracin, kanamycin, neomycin, gentamycin, erythromycin, cephalosporins, geldanamycin, and analogs thereof. Examples of cephalosporins include cephalothin, cephapirin, cefazolin, cephalexin, cephradine, cefadroxil, cefamandole, cefoxitin, cefaclor, cefuroxime, cefonicid, ceforanide, cefotaxime, moxalactam, ceftizoxime, ceftriaxone, and cefoperazone.

Antiseptics are recognized as substances that prevent or arrest the growth or action of microorganisms, generally in a nonspecific fashion, e.g., by inhibiting their activity or destroying them. Examples of antiseptics include silver sulfadiazine, chlorhexidine, glutaraldehyde, peracetic acid, sodium hypochlorite, phenols, phenolic compounds, iodophor compounds, quaternary ammonium compounds, and chlorine compounds.

Anti-viral agents are substances capable of destroying or suppressing the replication of viruses. Examples of anti-viral agents include α-methyl-P-adamantane methylamine, hydroxy-ethoxymethylguanine, adamantanamine, 5-iodo-2'-deoxyuridine, trifluorothymidine, interferon, and adenine arabinoside.

Enzyme inhibitors are substances that inhibit an enzymatic reaction. Examples of enzyme inhibitors include edrophonium chloride, N-methylphysostigmine, neostigmine bromide, physostigmine sulfate, tacrine HCl, tacrine, 1-hydroxymaleate, iodotubercidin, p-bromotetramisole, 10-(α-diethylaminopropionyl)-phenothiazine hydrochloride, calmidazolium chloride, hemicholinium-3, 3,5-dinitrocatechol, diacylglycerol kinase inhibitor I, diacylglycerol kinase inhibitor II, 3-phenylpropargylamine, N-monomethyl-L-arginine acetate, carbidopa, 3-hydroxybenzylhydrazine HCl, hydralazine HCl, clorgyline HCl, deprenyl HCl, L(-), deprenyl HCl, D(+), hydroxylamine HCl, iproniazid phosphate, 6-MeO-tetrahydro-9H-pyrido-indole, nialamide, pargyline HCl, quinacrine HCl, semicarbazide HCl, tranylcypromine HCl, N,N-diethylaminoethyl-2,2-diphenylvalerate hydrochloride, 3-isobutyl-1-methylxanthine, papaverine HCl, indomethacin, 2-cyclooctyl-2-hydroxyethylamine hydrochloride, 2, 3-dichloro-α-methylbenzylamine (DCMB), 8,9-dichloro-2,3,4,5-tetrahydro-1H-2-benzazepine hydrochloride, p-aminoglutethimide, p-aminoglutethimide tartrate, R(+), p-aminoglutethimide tartrate, S(-), 3-iodotyrosine, alpha-methyltyrosine, L(-) alpha-methyltyrosine, D L(-), cetazolamide, dichlorphenamide, 6-hydroxy-2-benzothiazolesulfonamide, and allopurinol.

Anti-pyretics are substances capable of relieving or reducing fever. Anti-inflammatory agents are substances capable of counteracting or suppressing inflammation. Examples of such agents include aspirin (salicylic acid), indomethacin, sodium indomethacin trihydrate, salicylamide, naproxen, colchicine, fenoprofen, sulindac, diflunisal, diclofenac, indoprofen and sodium salicylamide. Local anesthetics are substances that have an anesthetic effect in a localized region. Examples of such anesthetics include procaine, lidocaine, tetracaine and dibucaine.

Cell response modifiers are chemotactic factors such as platelet-derived growth factor (pDGF). Other chemotactic factors include neutrophil-activating protein, monocyte chemoattractant protein, macrophage-inflammatory protein, SIS (small inducible secreted) proteins, platelet factor, platelet basic protein, melanoma growth stimulating activity, epidermal growth factor, transforming growth factor (alpha), fibroblast growth factor, platelet-derived endothelial cell growth factor, insulin-like growth factor, nerve growth factor, and bone growth/cartilage-inducing factor (alpha and beta). Other cell response modifiers are the interleukins, interleukin inhibitors or interleukin receptors, including interleukin 1 through interleukin 10; interferons, including alpha, beta and gamma; hematopoietic factors, including erythropoietin, granulocyte colony stimulating factor, macrophage colony stimulating factor and granulocyte-macrophage colony stimulating factor; tumor necrosis factors, including alpha and beta; transforming growth factors (beta), including beta-1, beta-2, beta-3, inhibin, activin, and DNA that encodes for the production of any of these proteins.

Examples of statins include lovastatin, pravastatin, simvastatin, fluvastatin, atorvastatin, cerivastatin, rousvastatin, and superstatin.

Imaging agents are agents capable of imaging a desired site, e.g., tumor, *in vivo,* can also be included in the composition. Examples of imaging agents include substances having a label which is detectable in vivo, e.g., antibodies attached to fluorescent labels. The term antibody includes whole antibodies or fragments thereof.

Exemplary ligands or receptors include antibodies, antigens, avidin, streptavidin, biotin, heparin, type IV collagen, protein A, and protein G.

Exemplary antibiotics include antibiotic peptides.

In some aspects the bioactive agent can be selected to improve the compatibility (for example, with blood and/or surrounding tissues) of medical device surfaces. These agents, referred to herein as "biocompatible agents," when associated with the medical device surface, can serve to shield the blood from the underlying medical device material. Suitable biocompatible agents preferably reduce the likelihood for blood components to adhere to the medical device, thus reducing the formation of thrombus or emboli (blood clots that release and travel downstream).

The bioactive agent can provide antirestenotic effects, such as antiproliferative, antiplatelet, and/or antithrombotic effects. In some embodiments, the bioactive agent can include anti-inflammatory agents, immunosuppressive agents, cell attachment factors, receptors, ligands, growth factors, antibiotics, enzymes, nucleic acids, and the like. Compounds having antiproliferative effects include, for example, actinomycin D, angiopeptin, c-myc antisense, paclitaxel, taxane, and the like.

Representative examples of bioactive agents having antithrombotic effects include heparin, heparin derivatives, sodium heparin, low molecular weight heparin, hirudin, lysine, prostaglandins, argatroban, forskolin, vapiprost, prostacyclin and prostacyclin analogs, D-phenylalanyl-L-prolyl-L-arginyl-chloromethylketone (synthetic antithrombin), dipyridamole, glycoprotein IIb/IIIa platelet membrane receptor antibody, coprotein IIb/IIIa platelet membrane receptor antibody, recombinant hirudin, thrombin inhibitor (such as commercially available from Biogen), chondroitin sulfate, modified dextran, albumin, streptokinase, tissue plasminogen activator (TPA), urokinase, nitric oxide inhibitors, and the like.

The bioactive agent can also be an inhibitor of the GPIIb-IIIa platelet receptor complex, which mediates platelet aggregation. GPIIb/IIIa inhibitors can include monoclonal antibody Fab fragment c7E3, also know as abciximab (ReoPro™), and synthetic peptides or peptidomimetics such as eptifibatide (Integrilin^{™}) or tirofiban (Agrastat™).

The bioactive agent can be an immunosuppressive agent, for example, cyclosporine, CD-34 antibody, everolimus, mycophenolic acid, sirolimus, tacrolimus, and the like.

Other exemplary therapeutic antibodies include trastuzumab (Herceptin™), a humanized anti-HER2 monoclonal antibody (moAb); alemtuzumab (Campath™), a humanized anti-CD52 moAb; gemtuzumab (Mylotarg™), a humanized anti-CD33 moAb; rituximab (Rituxan™), a chimeric anti-CD20 moAb; ibritumomab (Zevalin™), a murine moAb conjugated to a beta-emitting radioisotope; tositumomab (Bexxar™), a murine anti-CD20 moAb; edrecolomab (Panorex™), a murine anti-epithelial cell adhesion molecule moAb; cetuximab (Erbitux™), a chimeric anti-EGFR moAb; and bevacizumab (Avastin™), a humanized anti-VEGF moAb.

Additionally, the bioactive agent can be a surface adhesion molecule or cell-cell adhesion molecule. Exemplary cell adhesion molecules or attachment proteins (such as extracellular matrix proteins including fibronectin, laminin, collagen, elastin, vitronectin, tenascin, fibrinogen, thrombospondin, osteopontin, von Willibrand Factor, bone sialoprotein (and active domains thereof), or a hydrophilic polymer such as hyaluronic acid, chitosan or methyl cellulose, and other proteins, carbohydrates, and fatty acids. Exemplary cell-cell adhesion molecules include N-cadherin and P-cadherin and active domains thereof.

Exemplary growth factors include fibroblastic growth factors, epidermal growth factor, platelet-derived growth factors, transforming growth factors, vascular endothelial growth factor, bone morphogenic proteins and other bone growth factors, and neural growth factors.

The bioactive agent can be also be selected from mono-2-(carboxymethyl) hexadecanamidopoly (ethylene glycol)₂₀₀ mono-4-benzoylbenzyl ether, mono-3-carboxyheptadecanamidopoly (ethylene glycol)₂₀₀ mono-4-benzoylbenzyl ether, mono-2-(carboxymethyl) hexadecanamidotetra (ethylene glycol) mono-4-benzoylbenzyl ether, mono-3-carboxyheptadecanamidotetra (ethylene glycol) mono-4-benzoylbenzyl ether, N-[2-(4-benzoylbenzyloxy) ethyl]-2-(carboxymethyl) hexadecanamide, N-[2-(4-benzoylbenzyloxy)ethyl]-3-carboxyheptadecanamide, N-[12-(benzoylbenzyloxy) dodecyl]-2-(carboxymethyl) hexadecanamide, N-[12-(benzoylbenzyloxy) dodecyl]-3-carboxy-heptadecanamide, N-[3-(4-benzoylbenzamido) propyl]-2-(carboxymethyl) hexadecanamide, N-[3-(4-benzoylbenzamido) propyl]-3-carboxyheptadecanamide, N-(3-benzoylphenyl)-2-(carboxymethyl) hexadecanamide, N-(3-benzoylphenyl)-3-carboxyheptadecanamide, N-(4-benzoylphenyl)-2-(carboxymethyl) hexadecanamide, poly(ethylene glycol)₂₀₀ mono-15-carboxypentadecyl mono-4-benzoylbenzyl ether, and mono-15-carboxypentadecanamidopoly (ethylene glycol)₂₀₀ mono-4-benzoylbenzyl ether.

Additional examples of contemplated bioactive agents and/or bioactive agent include analogues of rapamycin ("rapalogs"), ABT-578 from Abbott, dexamethasone, betamethasone, vinblastine, vincristine, vinorelbine, poside, teniposide, daunorubicin, doxorubicin, idarubicin, anthracyclines, mitoxantrone, bleomycins, plicamycin (mithramycin), mitomycin, mechlorethamine, cyclophosphamide and its analogs, melphalan, chlorambucil, ethylenimines and methylmelamines, alkyl sulfonates-busulfan, nitrosoureas, carmustine (BCNU) and analogs, streptozocin, trazenes-dacarbazinine, methotrexate, fluorouracil, floxuridine, cytarabine, mercaptopurine, thioguanine, pentostatin, 2-chlorodeoxyadenosine, cisplatin, carboplatin, procarbazine, hydroxyurea, mitotane, estrogen, ticlopidine, clopidogrel, abciximab, breveldin, cortisol, cortisone, fludrocortisone, prednisone, prednisolone, 6U-methylprednisolone, triamcinolone, acetaminophen, etodalac, tolmetin, ketorolac, ibuprofen and derivatives, mefenamic acid, meclofenamic acid, piroxicam, tenoxicam, phenylbutazone, oxyphenthatrazone, nabumetone, auranofin, aurothioglucose, gold sodium thiomalate, azathioprine, mycophenolate mofetil; angiotensin receptor blockers; nitric oxide donors; and mTOR inhibitors.

Viral particles and viruses include those that may be therapeutically useful, such as those used for gene therapy, and also attenuated viral particles and viruses which can promote an immune response and generation of immunity. Useful viral particles include both natural and synthetic types. Viral particles include, but are not limited to, adenoviruses, baculoviruses, parvoviruses, herpesviruses, poxviruses, adeno-associated viruses, vaccinia viruses, and retroviruses.

Other bioactive agents that can be used for altering gene function include plasmids, phages, cosmids, episomes, and integratable DNA fragments, antisense oligonucleotides, antisense DNA and RNA, modified DNA and RNA, iRNA, ribozymes, siRNA, and shRNA.

Other bioactive agents include cells such as platelets, stem cells, T lymphocytes, B lymphocytes, acidophils, adipocytes, astrocytes, basophils, hepatocytes, neurons, cardiac muscle cells, chondrocytes, epithelial cells, dendrites, endrocrine cells, endothelial cells, eosinophils, erythrocytes, fibroblasts, follicular cells, ganglion cells, hepatocytes, endothelial cells, Leydig cells, parenchymal cells, lymphocytes, lysozyme-secreting cells, macrophages, mast cells, megakaryocytes, melanocytes, monocytes, myoid cells, neck nerve cells, neutrophils, oligodendrocytes, oocytes, osteoblasts, osteochondroclasts, osteoclasts, osteocytes; plasma cells, spermatocytes, reticulocytes, Schwann cells, Sertoli cells, skeletal muscle cells, and smooth muscle cells. Bioactive agents can also include genetically modified, recombinant, hybrid, mutated cells, and cells with other alterations.

Additives such as inorganic salts, BSA (bovine serum albumin), and inert organic compounds can be used to alter the profile of bioactive agent release, as known to those skilled in the art.

The concentration of the bioactive agent or agents dissolved or suspended in the composition can range from about 0.01 to about 90 percent, by weight, based on the weight of the final composition.

The particular bioactive agent, or combination of bioactive agents, can be selected depending upon one or more of the following factors: the application of the controlled delivery device, the medical condition to be treated, the anticipated duration of treatment, characteristics of the implantation site, the number and type of bioactive agents to be utilized, and the like.

A comprehensive listing of bioactive agents can be found in The Merck Index. Thirteenth Edition, Merck & Co. (2001). Bioactive agents are commercially available from Sigma Aldrich Fine Chemicals, Milwaukee, WI.

In some aspects of the invention, the bioactive agent can be used to promote thrombosis in association with the natural biodegradable polysaccharide-based matrix. The degree of thrombosis can be controlled by various factors, including, for example, the presence of one or more thrombosis-promoting bioactive agents. Suitable thrombotic agents are described herein.

In some aspects the thrombotic agent can be selected to have an affect on the blood and/or surrounding tissues that are in contact with the occlusion surface. In some cases the thrombotic agent is chosen for the ability to affect the ability of blood components to adhere to the occlusion. Thrombotic agents include thrombin, collagen (for example, (synthetic) recombinant human collagen (FibroGen, South San Francisco, CA)), ADP, or convulxin.

Other prothrombotic or procoagulant factors include platelet factors 1-4, platelet activating factor (acetyl glyceryl ether phosphoryl choline); P-selectin and von Willebrand Factor (vWF); tissue factor; plasminogen activator initiator-1; thromboxane; procoagulant thrombin-like enzymes including cerastotin and afaâcytin; phospholipase A₂; Ca²⁺-dependent lectins (C-type lectin); factors that bind glycoprotein receptors and induce aggregation including aggretin, rhodocytin, aggregoserpentin, triwaglerin, and equinatoxin; glycoprotein Ib agonists including mamushigin and alboaggregin; vWF interacting factors including botrocetin, bitiscetin, cerastotin, and ecarin.

Other factors, including protein factors, that are involved in the clotting cascade include coagulation factors I - XIII (for example, fibrinogen, prothrombin, tissue thromboplastin, calcium, proaccelerin (accelerator globulin), proconvertin (serum prothrombin conversion accelerator), antihemophilic factor, plasma thromboplastin component, Stuart factor (autoprothrombin C), plasma thromboplastin antecedent (PTA), Hageman factor, and fibrin-stabilizing factor (FSF, fibrinase, protransglutaminase)).

Some surface adhesion molecule or cell-cell adhesion molecules may also function to promote coagulation or thrombosis. Exemplary cell adhesion molecules or attachment proteins (such as extracellular matrix proteins) include fibronectin, laminin, collagen, elastin, vitronectin, tenascin, fibrinogen, thrombospondin, osteopontin, von Willebrand Factor, bone sialoprotein (and active domains thereof), or a hydrophilic polymer such as hyaluronic acid, chitosan or methyl cellulose, and other proteins, carbohydrates, and fatty acids. Exemplary cell-cell adhesion molecules include N-cadherin and P-cadherin and active domains thereof.

The particular thrombotic agent, or a combination of thrombotic agents with other bioactive agents, can be selected depending upon one or more of the following factors: the medical condition to be treated, the anticipated duration of treatment, characteristics of the implantation site, the number and type of thrombogenic/bioactive agents to be utilized, the chemical composition (such as amylose, selected additives, and the like), the extent of coupling and the like.

In some aspects of the invention, a microparticle is used to deliver the bioactive agent from the natural biodegradable polysaccharide-based matrix. The microparticles of the invention can comprise any three-dimensional structure that can be immobilized on a substrate in association with the matrix formed by the amylose polymer. The term "microparticle" is intended to reflect that the three-dimensional structure is very small but not limited to a particular size range, or not limited to a structure that has a particular shape. According to the invention, microparticles typically have a size in the range of 5 nm to 100 µm in diameter. Generally microparticles are spherical or somewhat spherical in shape, but can have other shapes as well. In preferred embodiments of the invention, the biodegradable microparticles have a size in the range of 100 nm to 20 µm in diameter, and even more preferable in the range of 400 nm to 20 µm in diameter.

The microparticle being "biodegradable" refers to the presence of one or more biodegradable materials in the microparticle. The biodegradable microparticles include at least a biodegradable material (such as a biodegradable polymer) and a bioactive agent. The biodegradable microparticles can gradually decompose and release bioactive agent upon exposure to an aqueous environment, such as body fluids.

The biodegradable microparticle can also include one or more biodegradable polymers. Examples of biodegradable polymers that can be included in the biodegradable microparticle include, for example, polylactic acid, poly(lactide-co-glycolide), polycaprolactone, polyphosphazine, polymethylidenemalonate, polyorthoesters, polyhydroxybutyrate, polyalkeneanhydrides, polypeptides, polyanhydrides, and polyesters, and the like.

Biodegradable polyetherester copolymers can be used. Generally speaking, the polyetherester copolymers are amphiphilic block copolymers that include hydrophilic (for example, a polyalkylene glycol, such as polyethylene glycol) and hydrophobic blocks (for example, polyethylene terephthalate). Examples of block copolymers include poly(ethylene glycol)-based and poly(butylene terephthalate)-based blocks (PEG/PBT polymer). Examples of these types of multiblock copolymers are described in, for example, U.S. Patent No. 5,980,948. PEG/PBT polymers are commercially available from Octoplus BV, under the trade designation PolyActive™.

Biodegradable copolymers having a biodegradable, segmented molecular architecture that includes at least two different ester linkages can also be used. The biodegradable polymers can be block copolymers (of the AB or ABA type) or segmented (also known as multiblock or random-block) copolymers of the (AB)ₙ type. These copolymers are formed in a two (or more) stage ring opening copolymerization using two (or more) cyclic ester monomers that form linkages in the copolymer with greatly different susceptibilities to transesterification. Examples of these polymers are described in, for example, in U.S. Patent No. 5,252,701 (Jarrett et al., "Segmented Absorbable Copolymer").

Other suitable biodegradable polymer materials include biodegradable terephthalate copolymers that include a phosphorus-containing linkage. Polymers having phosphoester linkages, called poly(phosphates), poly(phosphonates) and poly(phosphites), are known. See, for example, Penczek et al., Handbook of Polymer Synthesis, Chapter 17: "Phosphorus-Containing Polymers," 1077-1132 (Hans R. Kricheldorf ed., 1992), as well as U.S. Patent Nos. 6,153,212, 6,485,737, 6,322,797, 6,600,010, 6,419,709. Biodegradable terephthalate polyesters can also be used that include a phosphoester linkage that is a phosphite. Suitable terephthalate polyester-polyphosphite copolymers are described, for example, in U.S. patent No. 6,419,709 (Mao et al., "Biodegradable Terephthalate Polyester-Poly(Phosphite) Compositions, Articles, and Methods of Using the Same). Biodegradable terephthalate polyester can also be used that include a phosphoester linkage that is a phosphonate. Suitable terephthalate polyester-poly(phosphonate) copolymers are described, for example, in U.S. Patent Nos. 6,485,737 and 6,153,212 (Mao et al., "Biodegradable Terephthalate Polyester-Poly(Phosphonate) Compositions, Articles and Methods of Using the Same). Biodegradable terephthalate polyesters can be used that include a phosphoester linkage that is a phosphate. Suitable terephthalate polyester-poly(phosphate) copolymers are described, for example, in U.S. Patent Nos. 6,322,797 and 6,600,010 (Mao et al., "Biodegradable Terephthalate Polyester-Poly(Phosphate) Polymers, Compositions, Articles, and Methods for Making and Using the Same).

Biodegradable polyhydric alcohol esters can also be used (See U.S. Patent No. 6,592,895). This patent describes biodegradable star-shaped polymers that are made by esterifying polyhydric alcohols to provide acyl moieties originating from aliphatic homopolymer or copolymer polyesters. The biodegradable polymer can be a three-dimensional crosslinked polymer network containing hydrophobic and hydrophilic components which forms a hydrogel with a crosslinked polymer structure, such as that described in U.S. Patent No. 6,583,219. The hydrophobic component is a hydrophobic macromer with unsaturated group terminated ends, and the hydrophilic polymer is a polysaccharide containing hydroxy groups that are reacted with unsaturated group introducing compounds. The components are convertible into a one-phase crosslinked polymer network structure by free radical polymerization. In yet further embodiments, the biodegradable polymer can comprise a polymer based upon α-amino acids (such as elastomeric copolyester amides or copolyester urethanes, as described in U.S. Patent No. 6,503,538).

The biodegradable microparticle can include one or more biodegradable polymers obtained from natural sources. In some preferred aspects the biodegradable polymer is selected from hyaluronic acid, dextran, starch, amylose, amylopectin, cellulose, xanthan, pullulan, chitosan, pectin, inulin, alginates, and heparin. One, or combinations of more than one of these biodegradable polymers, can be used. A particular biodegradable polymer can also be selected based on the type of bioactive agent that is present in the microparticle. Therefore, in some aspects of the invention, the biodegradable matrix can include a natural biodegradable polysaccharide matrix and a natural biodegradable polysaccharide-containing microparticle.

Therefore, in some embodiments, the microparticles include a natural biodegradable polysaccharide such as amylose or maltodextrin. In some embodiments the natural biodegradable polysaccharide can be the primary biodegradable component in the microparticle. In some embodiments, both the matrix and the microparticle include amylose and/or maltodextrin as components.

Dextran-based microparticles can be particularly useful for the incorporation of bioactive agents such as proteins, peptides, and nucleic acids. Examples of the preparation of dextran-based microparticles are described in U.S. Patent No. 6,303,148.

The preparation of amylose and other starch-based microparticles have been described in various references, including, for example, U.S. Patent No. 4,713,249; U.S. Patent No. 6,692,770; and U.S. Patent No. 6,703,048. Biodegradable polymers and their synthesis have been also been described in various references including Mayer, J.M., and Kaplan, D.L. (1994) Trends in Polymer Science 2:pages 227-235; and Jagur-Grodzinski, J., (1999) Reactive and Functional Polymers: Biomedical Application of Functional Polymers, Vol. 39, pages 99-138.

In some aspects of the invention, the biodegradable microparticle contains a biologically active agent (a "bioactive agent"), such as a pharmaceutical or a prodrug. Microparticles can be prepared incorporating various bioactive agents by established techniques, for example, by solvent evaporation (see, for example, Wichert, B. and Rohdewald, P. J Microencapsul. (1993) 10:195). The bioactive agent can be released from the biodegradable microparticle (the microparticle being present in the natural biodegradable polysaccharide matrix) upon degradation of the biodegradable microparticle *in vivo.* Microparticles having bioactive agent can be formulated to release a desired amount of the agent over a predetermined period of time. It is understood that factors affecting the release of the bioactive agent and the amount released can be altered by the size of the microparticle, the amount of bioactive agent incorporated into the microparticle, the type of degradable material used in fabricating the microparticle, the amount of biodegradable microparticles immobilized per unit area on the substrate, etc.

The microparticles can also be treated with a porogen, such as salt, sucrose, PEG, or an alcohol, to create pores of a desired size for incorporation of the bioactive agent.

The quantity of bioactive agents provided in the biodegradable microparticle can be adjusted by the user to achieve the desired effect. Biologically active compounds can be provided by the microparticles in a range suitable for the application. In another example, protein molecules can be provided by biodegradable microparticles. For example, the amount of protein molecules present can be in the range of 1-250,000 molecules per 1 µm diameter microparticle.

Generally, the concentration of the bioactive agent present in the biodegradable microparticles can be chosen based on any one or a combination of a number of factors, including, but not limited to, the release rate from the matrix, the type of bioactive agent(s) in the matrix, the desired local or systemic concentration of the bioactive agent following release, and the half life of the bioactive agent. In some cases the concentration of bioactive agent in the microparticle can be about 0.001% or greater, or in the range of about 0.001% to about 50 percent, or greater, by weight, based on the weight of the microparticle.

The particular bioactive agent to be included in the biodegradable microparticle, or combination of bioactive agents in microparticles, can be selected depending upon factors such as the application of the coated device, the medical condition to be treated, the anticipated duration of treatment, characteristics of the implantation site, the number and type of bioactive agents to be utilized, the chemical composition of the microparticle, size of the microparticle, crosslinking, and the like.

Biodegradable microparticles can be prepared having compositions that are suitable for either hydrophobic or hydrophilic drugs. For example, polymers such as polylactide or polycaprolactone can be useful for preparing biodegradable microparticles that include hydrophobic drugs; whereas polymers such as amylose or glycolide can be useful for preparing microparticles that include hydrophilic drugs.

In preferred aspects of the following methods, the natural biodegradable polysaccharide is selected from the group of amylose and maltodextrin. In other preferred aspects of the following methods, the natural biodegradable polysaccharide has a molecular weight of 500,000 Da or less, 250,000 Da or less, 100,000 Da or less, or 50,000 Da or less. It is also preferred that the natural biodegradable polysaccharides have an average molecular weight of 500 Da or greater. A particularly preferred size range for the natural biodegradable polysaccharides is in the range of about 1000 Da to about 10,000 Da.

During the step of activating, a composition including the natural biodegradable polysaccharide and the bioactive agent are contacted with the redox initiator and the initiator is activated to promote the crosslinking of two or more natural biodegradable polysaccharides via their pendent polymerisable groups. In preferred aspects the natural biodegradable polysaccharide includes a polymerizable group, such as an ethylenically unsaturated group, and initiator is capable of initiating free radical polymerization of the polymerizable groups. These methods are used *in situ* to form matrices, wherein the composition is disposed in a subject, respectively, rather than on a surface.

The invention will be further described with reference to the following non-limiting Examples. Unless otherwise indicated, all percentages are by weight.

### Example 1

### Synthesis of acrylated-amylose

Amylose having polymerizable vinyl groups was prepared by mixing 0.75g of amylose (A0512; Aldrich) with 100 mL of methylsulfoxide (JT Baker) in a 250 mL amber vial, with stirring. After one hour, 2 mL of triethylamine (TEA; Aldrich) was added and the mixture was allowed to stir for 5 minutes at room temperature. Subsequently, 2 mL of glycidyl acrylate (Polysciences) was added and the amylose and glycidyl acrylate were allowed to react by stirring overnight at room temperature. The mixture containing the amylose-glycidyl acrylate reaction product was dialyzed for 3 days against DI water using continuous flow dialysis. The resultant acrylated-amylose (0.50g; 71.4% yield) was then lyophilized and stored desiccated at room temperature with protection from light.

### Example 2

### Synthesis of MTA-PAAm

A polymerization initiator was prepared by copolymerizing a methacrylamide having a photoreactive group with acrylamide.

A methacrylamide-oxothioxanthene monomer (N-[3-(7-Methyl-9-oxothioxanthene-3-carboxamido) propyl]methacrylamide (MTA-APMA)) was first prepared. N-(3-aminopropyl)methacrylamide hydrochloride (APMA), 4.53 g (25.4 mmol), prepared as described in U.S. Patent No. 5,858,653, Example 2, was suspended in 100 mL of anhydrous chloroform in a 250 mL round bottom flask equipped with a drying tube. 7-methyl-9-oxothioxanthene-3-carboxylic acid (MTA) was prepared as described in U.S. Patent No. 4,506,083, Example D. MTA-chloride (MTA-Cl) was made as described in U.S. Patent No. 6,007,833, Example 1. After cooling the slurry in an ice bath, MTA-Cl (7.69 g; 26.6 mmol) was added as a solid with stirring to the APMA-chloroform suspension. A solution of 7.42 mL (53.2 mmol) of TEA in 20 mL of chloroform was then added over a 1.5 hour time period, followed by a slow warming to room temperature. The mixture was allowed to stir 16 hours at room temperature under a drying tube. After this time, the reaction was washed with 0.1 N HCl and the solvent was removed under vacuum after adding a small amount of phenothiazine as an inhibitor. The resulting product was recrystallized from tetrahydrofuran (THF)/toluene (3/1) and gave 8.87 g (88.7% yield) of product after air drying. The structure of MTA-APMA was confirmed by NMR analysis.

MTA-APMA was then copolymerized with acrylamide in DMSO in the presence of 2-mercaptoethanol (a chain transfer agent), N,N,N',N'-tetramethyl-ethylenediamine (a cocatalyst), and 2,2'-azobis(2-methyl-propionitrile) (a free radical initiator) at room temperature. The solution was sparged with nitrogen for 20 minutes, sealed tightly, and incubated at 55°C for 20 hours. The solution was dialyzed for 3 days against DI water using continuous flow dialysis. The resultant MTA-PAAm was lyophilized, stored desiccated, and protected from light at room temperature.

### Example 3

### Preparation of 4-bromomethylbenzophenone (BMBP)

4-Methylbenzophenone (750 g; 3.82 moles) was added to a 5 liter Morton flask equipped with an overhead stirrer and dissolved in 2850 mL of benzene. The solution was then heated to reflux, followed by the dropwise addition of 610 g (3.82 moles) of bromine in 330 mL of benzene. The addition rate was approximately 1.5 mL/min and the flask was illuminated with a 90 watt (90 joule/sec) halogen spotlight to initiate the reaction. A timer was used with the lamp to provide a 10% duty cycle (on 5 seconds, off 40 seconds), followed in one hour by a 20% duty cycle (on 10 seconds, off 40 seconds). At the end of the addition, the product was analyzed by gas chromatography and was found to contain 71% of the desired 4-bromomethylbenzophenone, 8% of the dibromo product, and 20% unreacted 4-methylbenzophenone. After cooling, the reaction mixture was washed with 10 g of sodium bisulfite in 100 mL of water, followed by washing with 3X 200 mL of water. The product was dried over sodium sulfate and recrystallized twice from 1:3 toluene:hexane. After drying under vacuum, 635 g of 4-bromomethylbenzophenone was isolated, providing a yield of 60%, having a melting point of 112°C - 114°C. Nuclear magnetic resonance ("NMR") analysis (¹H NMR (CDCl₃)) was consistent with the desired product: aromatic protons 7.20-7.80 (m, 9H) and methylene protons 4.48 (s, 2H). All chemical shift values are in ppm downfield from a tetramethylsilane internal standard.

### Example 4

### Preparation of ethylenebis(4-benzoylbenzyldimethylammonium) dibromide

N,N,N',N'-Tetramethylethylenediamine (6 g; 51.7 mmol) was dissolved in 225 mL of chloroform with stirring. BMBP (29.15 g; 106.0 mmol), as described in Example 3, was added as a solid and the reaction mixture was stirred at room temperature for 72 hours. After this time, the resulting solid was isolated by filtration and the white solid was rinsed with cold chloroform. The residual solvent was removed under vacuum and 34.4 g of solid was isolated for a 99.7% yield, melting point 218°C - 220°C. Analysis on an NMR spectrometer was consistent with the desired product: ¹H NMR (DMSO-d₆) aromatic protons 7.20-7.80 (m, 18H), benzylic methylenes 4.80 (br. s, 4H), amine methylenes 4.15 (br. s, 4H), and methyls 3.15 (br. s, 12H).

### Comparative Example 1

### Formation of an amylose matrix on PET mesh

Acrylated-amylose (100 mg), as described in Example 1, was placed in an 8mL amber vial. Ethylenebis(4-benzoylbenzyldimethylammonium) dibromide (3 mg), as described in Example 5, 2 µl of 2-NVP, and 1 mL of 1X phosphate buffered saline (1X PBS) was added to the acrylated-amylose and mixed for two hours on a shaker at 37°C. The mixture (250 µl) was spread onto a 3 cm x 2 cm polyethylene terephthalate (PET) mesh substrate (41 µm monofil diameter; Goodfellow Cambridge Ltd., UK). The PET substrate with the applied amylose mixture was placed in a Dymax Lightweld PC-2 illumination system (Dymax Corp.; light intensity 6.5 *m*W/*cm²),* 15 cm from the light source, and illuminated for 60 seconds. After illumination, the applied amylose mixture was found to form a semi-firm gel on the PET substrate, with elastomeric properties evident.

### Example 5

### Preparation of 1-(6-oxo-6-hydroxyhexyl)maleimide (Mal-EACA)

A maleimide functional acid was prepared in the following manner, and was used in Example 7. EACA (6-aminocaproic acid), (100 g; 0.762 moles), was dissolved in 300 mL of acetic acid in a three-neck, three liter flask equipped with an overhead stirrer and drying tube. Maleic anhydride, (78.5 g; 0.801 moles), was dissolved in 200 mL of acetic acid and added to the EACA solution. The mixture was stirred one hour while heating on a boiling water bath, resulting in the formation of a white solid. After cooling overnight at room temperature, the solid was collected by filtration and rinsed two times with 50 mL of hexane each rinse. After drying, the yield of the (z)-4-oxo-5-aza-undec-2-endioic acid (Compound 1) was in the range of 158-165 g (90-95%) with a melting point of 160-165°C. Analysis on an NMR spectrometer was consistent with the desired product: ¹H NMR (DMSO-d₆, 400 MHz) 8 6.41, 6.24 (d, 2H, J = 12.6 Hz; vinyl protons), 3.6-3.2 (b, 1H; amide proton), 3.20-3.14 (m, 2H: methylene adjacent to nitrogen), 2.20 (t, 2H, J = 7.3; methylene adjacent to carbonyl), 1.53-1.44 (m, 4H; methylenes adjacent to the central methylene), and 1.32-1.26 (m, 2H; the central methylene).

(z)-4-oxo-5-aza-undec-2-endioic acid, (160 g; 0.698 moles), zinc chloride, 280 g (2.05 moles), and phenothiazine, 0.15 g were added to a two liter round bottom flask fitted with an overhead stirrer, condenser, thermocouple, addition funnel, an inert gas inlet, and heating mantle. Chloroform (CHCl₃), 320 mL was added to the 2 liter reaction flask, and stirring of the mixture was started. Triethylamine (480 mL; 348 g, 3.44 moles (TEA)) was added over one hour. Chlorotrimethyl silane (600 mL; 510 g, 4.69 moles) was then added over two hours. The reaction was brought to reflux and was refluxed overnight (∼16 hours). The reaction was cooled and added to a mixture of CHCl₃ (500 mL), water (1.0 liters), ice (300 g), and 12 N hydrochloric acid (240 mL) in a 20 liter container over 15 minutes. After 15 minutes of stirring, the aqueous layer was tested to make sure the pH was less than 5.. The organic layer was separated, and the aqueous layer was extracted three times with CHCl₃ (700 mL) each extraction. The organic layers were combined and evaporated on a rotary evaporator. The residue was then placed in a 20 liter container. A solution of sodium bicarbonate (192 g) in water (2.4 liters) was added to the residue. The bicarbonate solution was stirred until the solids were dissolved. The bicarbonate solution was treated with a solution of hydrochloric acid, (26 liters of 1.1 N) over 5 minutes to a pH of below 2. The acidified mixture was then extracted with two portions of CHCl₃, (1.2 liters and 0.8 liters) each extraction. The combined extracts were dried over sodium sulfate and evaporated. The residue was recrystallized from toluene and hexane. The crystalline product was then isolated by filtration and dried which produced 85.6 g of white N-(6-oxo-6-hydroxyhexyl)maleimide (Mal-EACA; Compound 2). Analysis on an NMR spectrometer was consistent with the desired product: ¹H NMR (CDCl₃, 400 MHz) δ 6.72 (s, 2H; maleimide protons), 3.52 (t, 2H, J = 7.2 Hz; methylene next to maleimide), 2.35 (t, 2H, J = 7.4; methylene next to carbonyl), 1.69 -1.57 (m, 4H; methylenes adjacent to central methylene), and 1.39 -1.30 (m, 2H; the central methylene). The product had a DSC (differential scanning calorimator) melting point peak at 89.9°C.

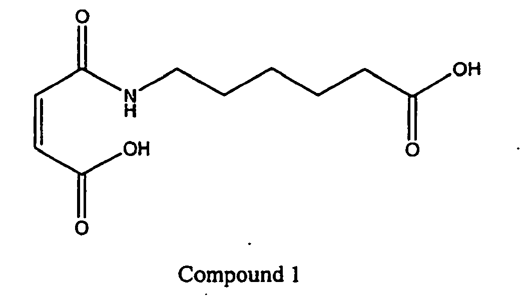

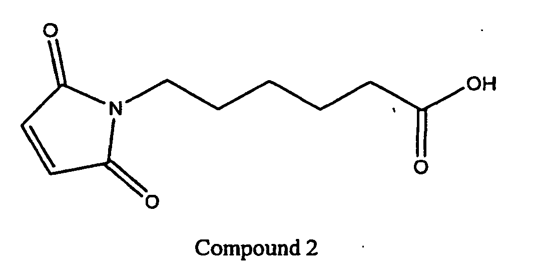

### Example 6

### Preparation of N-(5-isocyanatopentyl) maleimide (Mal-C5-NCO)

Mal-EACA from Example 6 (5.0 g; 23.5 mmole) and CHCl₃ (25 mL) were placed in a 100 mL round bottom flask and stirred using a magnetic bar with cooling in an ice bath. Oxalyl chloride (10.3 my; ∼15 g; 118 mmole) was added and the reaction was brought to room temperature with stirring overnight. The volatiles were removed on a rotary evaporator, and the residue was azetroped with three times with 10 mL CHCl₃ each time. The intermediate Mal-EAC-Cl [N-(6-oxo-6-chlorohexyl)maleimide] (Compound 3) was dissolved in acetone (10 mL) and added to a cold (ice bath) stirred solution of sodium azide (2.23 g; 34.3 mmole) in water (10 mL). The mixture was stirred one hour using an ice bath. The organic layer was set aside in an ice bath, and the aqueous layer was extracted three times with 10 mL CHCl₃. All operations of the acylazide were done at ice bath temperatures. The combined organic solutions of the azide reaction were dried for an hour over anhydrous sodium sulfate. The N-(6-oxo-6-azidohexyl)maleimide (Compound 4) solution was further dried by gentle swirling over molecular sieves over night. The cold azide solution was filtered and added to refluxing CHCl₃, 5 mL over a 10 minute period. The azide solution was refluxed for 2 hours. The weight of Mal-C5-NCO (Compound 5) solution obtained was 55.5 g, which was protected from moisture. A sample of the isocyanate solution, 136 mg was evaporated and treated with DBB (1,4-dibromobenzene), 7.54 mg and chloroform-d, 0.9 mL: ¹H NMR (CDCl₃, 400MHz) δ 6.72 (s,2H), 3.55 (t, 2H, J = 7.2 Hz), 3.32 (t, 2H, J = 6.6 Hz), 1.70-1.59 (m, 4H), 1.44-1.35 (m, 2H). The NMR spectra was consistent with desired product. The DBB internal standard δ at 7.38 (integral value was 2.0, 4H; per mole of product) was used to estimate the moles of Mal-C5-NCO in solution. The calculated amount of product in solution was 23.2 mmole for a yield of 98% of theory. NCO reagent (concentration was 0.42 nnnole/g) was used to prepare a macromer in Example 12.

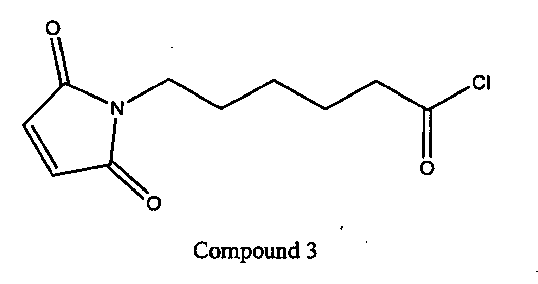

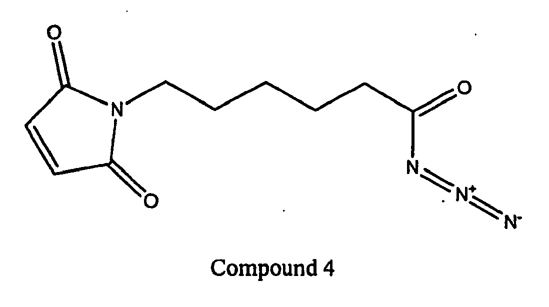

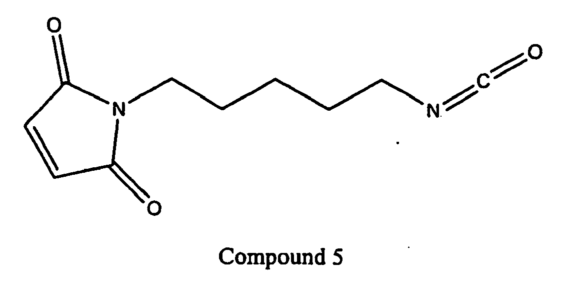

### Example 7

### Preparation of 3-(acryloyloxy)propanoic acid (2-carboxyethyl acrylate; CEA)

Acrylic acid (100 g; 1.39 mole) and phenothiazine (0.1 g) were placed in a 500 mL round bottom flask. The reaction was stirred at 92°C for 14 hours. The excess acrylic acid was removed on a rotary evaporator at 25°C using a mechanical vacuum pump. The amount of residue obtained was 51.3 g. The CEA (Compound 6) was used in Example 8 without purification.

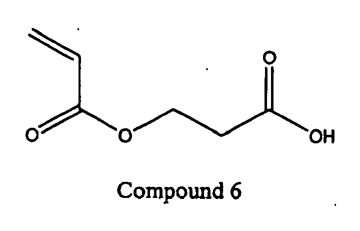

### Example 8

### Preparation of 3-chloro-3-oxopropyl acrylate (CEA-Cl)

CEA from Example 7 (51 g; ∼ 0.35 mole) and dimethyl formamide (DMF; 0.2 mL; 0.26 mmole) were dissolved in CH₂Cl₃ (100 mL). The CEA solution was added slowly (over 2 hours) to a stirred solution of oxalyl chloride (53 mL; 0.61 mole), DMF (0.2 mL; 2.6 mole), anthraquinone (0.5 g; 2.4 mmole), phenothiazine (0.1 g, 0.5 mmole), and CH₂Cl₃ (75 mL) in a 500 mL round bottom flask in an ice bath at 200 mm pressure. A dry ice condenser was used to retain the CH₂Cl₃ in the reaction flask. After the addition was complete the reaction was stirred at room temperature overnight. The weight of reaction solution was 369 g. A sample of the CEA-Cl (Compound 7) reaction solution (124 mg) was treated with 1,4-dibromobenzene (DBB, 6.85 mg) evaporated and dissolved in CDCl₃: ¹H NMR (CDCl₃, 400 MHz) δ 7.38 (s, 4H; DBB internal std.), 6.45 (d, 1H, J = 17.4 Hz), 6.13 (dd, 1H, J = 17.4, 10.4 Hz), 5.90 (d, I H, J = 10.4 Hz), 4.47 (t, 2H, J = 5.9 Hz), 3.28 (t, 2H, J = 5.9). The spectra was consistent with the desired product. There was 0.394 mole DBB for 1.0 mole CEA-Cl by integration, which gave a calculated yield of 61 %. Commercially available CEA (426 g; Aldrich) was reacted with oxalyl chloride (532 mL) in a procedure similar to the one listed above. The residue CEA-Cl (490 g) was distilled using an oil bath at 140°C at a pressure of 18 mm Hg. The distillate temperature reached 98°C and 150 g of distillate was collected. The distillate was redistilled at 18 mm Hg at a maximum bath temperature of 120°C. The temperature range for the distillate was 30°C to 70°C which gave 11 g of material. The distillate appeared to be 3-chloro-3-oxopropyl 3-chloropropanoate. The residue of the second distillation (125 g; 26 % of theory) was used in Example 9.

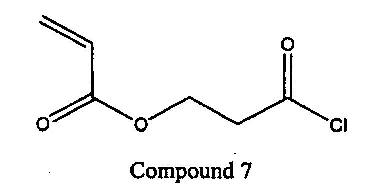

### Example 9

### Preparation of 3-azido-3-oxopropyl acrylate (CEA-N3)

CEA-Cl from Example 8 (109.2 g; 0.671 mole) was dissolved in acetone (135 mL). Sodium azide (57.2 g; 0.806 mole) was dissolved in water (135 mL) and chilled. The CEA-Cl solution was then added to the chilled azide solution with vigorous stirring in an ice bath for 1.5 hours. The reaction mixture was extracted two times with 150 mL of CHCl₃ each extraction. The CHCl₃ solution was passed through a silica gel column 40 mm in diameter by 127 mm. The 3-azido-3-oxopropyl acrylate (Compound 8) solution was gently agitated over dried molecular sieves at 4°C overnight. The dried solution was used in Example 10 without purification.

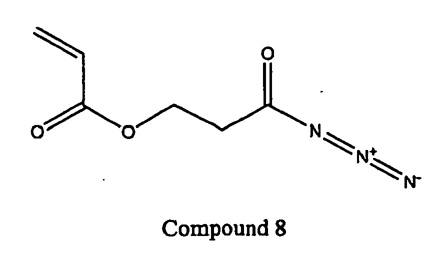

### Example 10

### Preparation of 2-isocyanatoethyl acrylate (EA-NCO)

The dried azide solution (from Example 9) was slowly added to refluxing CHCl₃, 75 mL. After the addition was completed, refluxing was continued 2 hours. The EA-NCO (Compound 9) solution (594.3 g) was protected from moisture. A sample of the EA-NCO solution (283.4 mg) was mixed with DBB (8.6 mg) and evaporated. The residue was dissolved in CDCl₃: ¹H NMR (CDCl₃, 400 MHz) δ 7.38 (s, 4H; DBB internal std.), 6.50 (d, 1H, J = 17.3 Hz), 6.19 (dd, 1H, J = 17.3, 10.5 Hz), 5.93 (d, I H, J = 10.5 Hz), 4.32 (t, 2H, J = 5.3 Hz), 3.59 (t, 2H, J = 5.3). The spectra was consistent with the desired EA-NCO. There was 0.165 mole DBB for 1.0 mole EA-NCO by integration, which gave a calculated concentration of 110 mg EA-NCO/g of solution. The EA-NCO solution was used to prepare a macromer in Example 11.

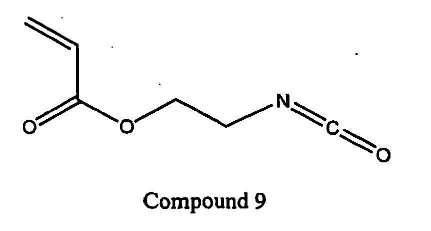

### Example 11

### Preparation of Maltodextrin-acrylate macromer (MD-Acrylate)

Maltodextrin (MD; Aldrich; 9.64 g; ∼3.21 mmole; DE (Dextrose Equivalent): 4.0 - 7.0) was dissolved in dimethylsulfoxide (DMSO) 60 mL. The size of the maltodextrin was calculated to be in the range of 2,000 Da - 4,000 Da. A solution of EA-NCO from Example 12 (24.73 g; 19.3 mmole) was evaporated and dissolved in dried DMSO (7.5 mL). The two DMSO solutions were mixed and heated to 55°C overnight. The DMSO solution was placed in dialysis tubing (1000 MWCO, 45 mm flat width x 50 cm long) and dialyzed against water for 3 days. The macromer solution was filtered and lyophilized to give 7.91 g white solid. A sample of the macromer (49 mg), and DBB (4.84 mg) was dissolved in 0.8 mL DMSO-d₆: ¹H NMR (DMSO-d₆, 400 MH₂) δ 7.38 (s, 4H; internal std. integral value of 2.7815), 6.50, 6.19, and 5.93 (doublets, 3H; vinyl protons integral value of 3.0696). The calculated acrylate load of macromer was 0.616 µmoles/mg of polymer.

### Example 12

### Preparation of Maltodextrin-maleimide macromer (MD-Mal)

A procedure similar to Example 11 was used to make the MD-Mal macromer. A solution of Mal-C5-NCO from Example 8 (0.412 g; 1.98 mmole) was evaporated and dissolved in dried DMSO (2 mL). MD (0.991 g; 0.33 mmole) was dissolved in DMSO (5 mL). The DMSO solutions were combined and stirred at 55°C for 16 hours. Dialysis and lyophilization gave 0.566 g product. A sample of the macromer (44 mg), and DBB (2.74 mg) was dissolved in 00.8 mL DMSO-d₆: ¹H NMR (DMSO-d₆, 400 MHz) δ 7.38 (s, 4H; internal std. integral value of 2.3832), 6.9 (s, 2H; Maleimide protons integral value of 1.000). The calculated acrylate load of macromer was 0.222 µmoles/mg of polymer. The macromer was tested for its ability to make a matrix (see Comparative Example 5)

### Comparative Example 2

### Formation of Maltodextrin-acrylate biodegradable matrix using MTA-PAAm

250 mg of MD-Acrylate as prepared in Example 11 was placed in an 8 mL amber vial. To the MD-Acrylate was added 3 mg of MTA-PAAm (lyophilized), 2 µL of 2-NVP, and 1 mL of 1X phosphate-buffered saline (1X PBS), providing a composition having MD-Acrylate at 250 mg/mL. The reagents were then mixed for one hour on a shaker at 37°C. The mixture in an amount of 50 µL was placed onto a glass slide and illuminated for 40 seconds with an EFOS 100 SS illumination system equipped with a 400-500 nm filter. After illumination the polymer was found to form a semi-firm gel having elastomeric properties.

### Comparative Example 3

### Formation of MD-Acrylate biodegradable matrix using camphorquinone

250 mg ofMD-acrylate as prepared in Example 11 was placed in an 8mL amber vial. To the MD-Acrylate was added 14 mg of camphorquinone-10-sulfonic acid hydrate (Toronto Research Chemicals, Inc.), 3 µL of 2-NVP, and 1 mL of distilled water. The reagents were then mixed for one hour on a shaker at 37°C. The mixture in an amount of 50 µL was placed onto a glass slide and illuminated for 40 seconds with a SmartliteIQ^{™} LED curing light (Dentsply Caulk). After illumination the polymer was found to form a semi-firm gel having with elastomeric properties.

### Comparative Example 4

### Formation of MD-Mal biodegradable matrix using MTA-PAAm

250 mg of MD-Mal as prepared in Example 12 was placed in an 8 mL amber vial. To the MD-Mal was added 3 mg of MTA-PAAm (lyophilized), µL of 2-NVP, and 1 mL of 1X phosphate-buffered saline (1X PBS). The reagents were then mixed for one hour on a shaker at 37°C. The mixture in an amount of 50 µL was placed onto a glass slide and illuminated for 40 seconds with an EFOS 100 SS illumination system equipped with a 400-500 nm filter. After illumination the polymer was found to form a semi-firm gel having elastomeric properties.

### Comparative Example 5

### Bioactive agent incorporation/release from a MD-Acrylate Matrix

500 mg of MD-Acrylate as prepared in Example 11 was placed in an 8 mL amber vial. To the MD-Acrylate was added 3 mg of MTA-PAAm (lyophilized), 2 µL of 2-NVP, and 1 mL of 1X phosphate-buffered saline (1X PBS). The reagents were then mixed for one hour on a shaker at 37°C. To this mixture was added either 5 mg 70kD FITC-Dextran or 5mg 10kD FITC-Dextran (Sigma) and vortexed for 30 seconds. The mixture in an amount of 200 µL was placed into a Teflon well plate (8mm diameter, 4mm deep) and illuminated for 40 seconds with an EFOS 100 SS illumination system equipped with a 400-500 nm filter. The formed matrix was loose, and not as well crosslinked as the formed MD-acrylate matrix in Comparative Example 3. After illumination, the matrix was transferred to a 12 well plate (Falcon) and placed in a well containing 0.6 mL PBS. At daily intervals for 6 days, 150 µL of PBS was removed from each well and placed into a 96 well plate. The remaining 850 µL were removed from the samples, and replaced with 1 mL fresh PBS. The 96 well plate was analyzed for FITC-Dextran on a spectrophotometer (Shimadzu) at 490 absorbance. Results showed that at least 70% of the detectable 10kd or 70kD FITC-Dextran was released from the matrix after 2 days. Visual observation showed that an unquantified amount of 10 kD or 70kD FITC-Dextran remained within the matrix after 6 days.

### Example 13

### Polyalditol-acrylate synthesis

Polyalditol (PA; GPC; 9.64 g; ~3.21 mmole) was dissolved in dimethylsulfoxide (DMSO) 60 mL. The size of the polyalditol was calculated to be in the range of 2,000 Da - 4,000 Da. A solution of EA-NCO from Example 11 (24.73 g; 19.3 mmole) was evaporated and dissolved in dried DMSO (7.5 mL). The two DMSO solutions were mixed and heated to 55°C overnight. The DMSO solution was placed in dialysis tubing (1000 MWCO, 45 mm flat width x 50 cm long) and dialyzed against water for 3 days. The polyalditol macromer solution was filtered and lyophilized to give 7.91 g white solid. A sample of the macromer (49 mg), and DBB (4.84 mg) was dissolved in 0.8 mL DMSO-d₆: ¹H NMR (DMSO-d₆, 400 MHz) δ 7.38 (s, 4H; internal std. integral value of 2.7815), 6.50, 6.19, and 5.93 (doublets, 3H; vinyl protons integral value of 3.0696). The calculated acrylate load of macromer was 0.616 µmoles/mg of polymer.

### Example 14

### Formation of a Maltodextrin-acrylate biodegradable matrix using REDOX chemistry

Two solutions were prepared. Solution #1 was prepared as follows: 250 mg of MD-acrylate as prepared in Example 12 was placed in an 8 mL vial. To the MD-acrylate was added 15 mg ferrous gluconate hydrate (Sigma), 30 mg Ascorbic Acid (Sigma), 67 uL AMPS (Lubrizol) and 1,000 uL deionized water. Solution #2 was prepared as follows: 250 mg of MD-acrylate as prepared in Example 12 was placed in a second 8 mL vial. To this MD-acrylate was added 30 uL AMPS, 80 uL Hydrogen Peroxide (Sigma) and 890 uL 0.1 M Acetate buffer (pH 5.5).

50 uL of Solution #1 was added to a glass slide. 50 uL of solution #2 was added to Solution #1 with slight vortexing. After mixing for 2 seconds, the mixture polymerized and formed a semi-firm gel having elastomeric properties.

### Example 15

### Formation of Maltodextrin-acrylate Biodegradable Matrix using REDOX Chemistry

Two solutions were prepared, similar to Example 26, but in this Example Solution #1 different concentrations of ferrous gluconate hydrate (Sigma) and ascorbic acid were used. Solution #1 was prepared as follows: 250 mg of MD-acrylate (as prepared in Example 12) was placed in an 8 mL vial. To the MD-acrylate was added 5 mg ferrous gluconate hydrate (Sigma), 40 mg ascorbic acid (Sigma), 67 uL AMPS (Lubrizol) and 1,000 uL deionized water. Solution #2 was prepared as follows: 250 mg of MD-acrylate as prepared in Example 6 was placed in a second 8 mL vial. To this MD-acrylate was added 30 uL AMPS, 80 uL Hydrogen Peroxide (Sigma) and 890 uL 0.1 M Acetate buffer (pH 5.5).

50 uL of Solution #1 was added to a glass slide. 50 uL of solution #2 was added to Solution #1 with slight vortexing. After mixing for 8 seconds, the mixture polymerized and formed a semi-firm gel having elastomeric properties.

### Example 16

### Formation of Maltodextrin-acrylate Biodegradable Matrix using REDOX Chemistry

Two solutions were prepared. Solution #1 was prepared as follows: 250 mg of MD-acrylate (as prepared in Example 12) was placed in an 8 mL vial. To the MD-acrylate was added 15 mg Iron (II) L-Ascorbate (Sigma), 30 mg Ascorbic Acid (Sigma), 67 uL AMPS (Lubrizol) and 1,000 uL deionized water. Solution #2 was prepared as follows: 250 mg of MD-acrylate as prepared in Example 6 was placed in a second 8 mL vial. To this MD-acrylate was added 30 uL AMPS, 80 uL hydrogen peroxide (Sigma) and 890 uL 0.1 M Acetate buffer (pH 5.5).

50 uL of Solution #1 was added to a glass slide. 50 uL of solution #2 was added to Solution #1 with slight vortexing. After mixing for 2 seconds, the mixture polymerized and formed a semi-firm gel having elastomeric properties.

### Example 17

### Formation of Polyalditol-acrylate biodegradablematrix using REDOX chemistry

Two solutions were prepared. Solution #1 was prepared as follows: 1,000 mg of Polyalditol-acrylate as prepared in Example 16 was placed in an 8 mL vial. To the Polyalditol-acrylate was added 15 mg Ferrous Sulfate Heptahydrate (Sigma), 30 mg Ascorbic Acid (Sigma), 67 uL AMPS (Lubrizol) and 1,000 uL deionized water; Solution #2 was prepared as follows: 1,000 mg of Polyalditol-acrylate as prepared in Example 13 was placed in a second 8 mL vial. To this Polyalditol-acrylate was added 30 uL AMPS, 80 uL Hydrogen Peroxide (Sigma) and 890 uL 0.1 M Acetate buffer (pH 5.5).

50 uL of Solution #1 was added to a glass slide. 50 uL of solution #2 was added to Solution #1 with slight vortexing. After mixing for 2 seconds, the mixture polymerized and formed a semi-firm gel having elastomeric properties.

### Example 18

### Bioactive agent incorporation into a MD-Acrylate Matrix

Two solutions were prepared. Solution #1 was prepared as follows: 250 mg of MD-acrylate (as prepared in Example 12) was placed in an 8 ml vial. To the MD-acrylate was added 15 mg Iron (II) Acetate (Sigma), 30 mg Ascorbic Acid (Sigma), 67 ul AMPS (Lubrizol), 75 mg Bovine Serum Albumin (BSA; representing the bioactive agent) and 1,000 µL deionized water. Solution #1 was prepared as follows: 250 mg of MD-acrylate was placed in a second 8 ml vial. To this MD-acrylate was added 30 µL, AMPS, 80 µL Hydrogen Peroxide (Sigma), 75 mg BSA and 890 µL, Acetate buffer (pH 5.5).

50 µL of Solution #1 was added to a glass slide. 50 µL of solution #2 was added to Solution #1 with slight vortexing. After mixing for 2 seconds, the mixture polymerized and formed a semi-firm gel having elastomeric properties.

### Example 19

### Preparation of Acylated Maltodextrin (Butyrylated-MD)

Maltodextrin having pendent butyryl groups were prepared by coupling butyric anhydride at varying molar ratios.

To provide butyrylated-MD (1 butyl/4 glucose units, 1:4 B/GU) the following procedure was performed. Maltodextrin (MD; Aldrich; 11.0 g; 3.67 mmole; DE (Dextrose Equivalent): 4.0 - 7.0) was dissolved in dimethylsulfoxide (DMSO) 600 mL with stirring. The size of the maltodextrin was calculated to be in the range of 2,000 Da - 4,000 Da. Once the reaction solution was complete, 1-methylimidazole (Aldrich; 2.0g, 1.9mls) and butyric anhydride (Aldrich; 5.0 g, 5.2 mls) was added with stirring. The reaction mixture was stirred for four hours at room temperature. After this time, the reaction mixture was quenched with water and dialyzed against DI water using 1,000 MWCO dialysis tubing. The butyrylated starch was isolated via lyophylization to give 9.315 g (85 % yield). NMR confirmed a butyrylation of 1:3 B/GU (1.99mmoles butyl/g sample).

To provide butyrylated-MD (1:8 B/GU), 2.5g (2.6 mL) butyric anhydride was substituted for the amount of butyric anhydride described above: A yield of 79% (8.741 g) was obtained. NMR confirmed a butyrylation of 1:5 B/GU (1.31 mmoles butyl/g sample).

To provide butyrylated-MD (1:2B/GU), 10.0g (10.4 mL) butyric anhydride was substituted for the amount of butyric anhydride described above. A yield of 96% (10.536 g) was obtained. NMR confirmed a butyrylation of 1:2 B/GU (3.42 mmoles butyl/g sample).

### Example 20

### Preparation of Acrylated Acylated Maltodextrin (Butyrylated-MD-Acrylate)

Preparation of an acylated maltodextrin macromer having pendent butyryl and acrylate groups prepared by coupling butyric anhydride at varying molar ratios.

To provide butyrylated-MD-acrylate (1 butyl/4 glucose units, 1:4 B/GU) the following procedure was performed. MD-Acrylate (Example 13; 1.1 g; 0.367 mmoles) was dissolved in dimethylsulfoxide (DMSO) 60 mL with stirring. Once the reaction solution was complete, 1-methylimidazole (0.20g, 0.19mls) and butyric anhydride (0.50 g, 0.52 mls) was added with stirring. The reaction mixture was stirred for four hours at room temperature. After this time, the reaction mixture was quenched with water and dialyzed against DI water using 1,000 MWCO dialysis tubing. The butyrylated starch acrylate was isolated via lyophylization to give 821 mg (75% yield, material lost during isolation). NMR confirmed a butyrylation of 1:3 B/GU (2.38 mmoles butyl/g sample).

### Example 21

### Preparation of Acrylated Acylated Maltodextrin (Bulyrylated-N4D-Aclylate)

Maltodextrin having pendent butyryl and acrylate groups prepared by coupling butyric anhydride at varying molar ratios.

To provide butyrylated-MD-acrylate the following procedure is performed. Butyrylated-MD (Example 43; 1.0 g; 0.333 mmole) is dissolved in dimethylsulfoxide (DMSO) 60 mL with stirring. Once the reaction solution is complete, a solution of EA-NCO from Example 12 (353 mg; 2.50 mmole) is evaporated and dissolved in dried DMSO 1.0 mL). The two DMSO solutions are mixed and heated to 55°C overnight. The DMSO solution is placed in dialysis tubing (1000 MWCO) and dialyzed against water for 3 days. The macromer solution is filtered and lyophilized to give a white solid.

### Example 22

### Preparation of Maltodextrin-methacrylate macromer (MD-methacrylate)

To provide MD-methacrylate, the following procedure was performed. Maltodextrin (MD; Aldrich; 100 g; 3.67 mmole; DE: 4.0 - 7.0) was dissolved in dimethylsulfoxide (DMSO) 1,000 mL with stirring. The size of the maltodextrin was calculated to be in the range of 2,000 Da - 4,000 Da. Once the reaction solution was complete, 1-methylimidazole (Aldrich; 2.0g, 1.9mL) followed by methacrylic-anhydride (Aldrich; 38.5 g) were added with stirring. The reaction mixture was stirred for one hour at room temperature. After this time, the reaction mixture was quenched with water and dialyzed against DI water using 1,000 MWCO dialysis tubing. The MD-methacrylate was isolated via lyophylization to give 63.283 g (63 % yield). The calculated methacrylate load of macromer was 0.33 µmoles/mg of polymer

### Example 23

### Formation of a MD-methacrylate biodegradable matrix using REDOX chemistry

Two solutions were prepared. Solution #1 was prepared as follows: 250 mg of MD-methacrylate as prepared in example 22 was placed in an 8 mL vial. To the MD-methacrylate was added 9 mg ferrous gluconate hydrate (Sigma), 30 mg ascorbic acid (Sigma), and 1,000 uL deionized water. Solution #2 was prepared as follows: 250 mg of MD-methacrylate as prepared in example 22 was placed in a second 8 mL vial. To this MD-methacrylate was added 80 uL hydrogen peroxide (Sigma) and 920 uL 0.1 M acetate buffer (pH 5.5).

50 uL of Solution #1 was added to a glass slide. 50 uL of solution #2 was added to Solution #1, with slight mixing. After mixing for 5 seconds, the mixture polymerized and formed a semi-firm gel having elastomeric properties.

### Example 24

### Formation of a MD-methacrylate biodegradable matrix using REDOX chemistry/microcatheter delivery system

MD-methacrylate redox compositions were prepared having variations in MD-methacrylate concentrations and redox components. These compositions were delivered via microcatheters to a target site where, upon mixing, matrix formation occurred. Table 2 shows results of the experiments.

Reductant and oxidant solutions including MD-acrylate (MD-A) at different concentrations were prepared (see table 10, rows A and B). Solutions 1A and 1B were prepared as follows: 250 mg or 500 mg of MD-acrylate (as prepared in Example 12) was placed in an 8 mL vial. To the MD-acrylate was added 10 mg iron (II) L-ascorbate (Sigma), 20 mg ascorbic acid (Sigma), and 1,000 uL deionized water. Solutions 2A and 2B were prepared as follows: 250 mg or 500 mg of MD-acrylate (as prepared in Example 12) was placed in a second 8 mL) vial. To this MD-acrylate was added 80 uL hydrogen peroxide (Sigma) and 920 uL 0.1 M acetate buffer (pH 5.5).

Reductant and oxidant solutions including NM-methacrylate (MD-MA) at different concentrations were prepared (see table 10, rows C-G). Solutions 1C-1G were prepared as follows: 250 mg, 350 mg, or 500 mg of MD-methacrylate (as prepared in example 47) were individually placed in an 8 mL vial. To the MD-methacrylate was added 10 mg iron (II) L-ascorbate (Sigma), 20 mg ascorbic acid (Sigma), and 1,000 uL deionized water. Solutions 2C-2G were prepared as follows: 250 mg, 350 mg, or 500 mg of MD-methacrylate (as prepared in Example 22) was placed in a second 8 mL vial. To this MD-methacrylate was added 80 uL hydrogen peroxide (Sigma) and 920 uL 0.1 M Acetate buffer (pH 5.5).

Solution 1 (individually, A-G) was added to a 3 mL syringe (Becton-Dickinson), and the syringe was attached to a microcatheter (Excelsior SL-10; 2.4-1.7 fr; Boston Scientific or Renegdae; 3.0-2.5 fr; Boston Scientific). Applying moderate pressure to the syringe, approximately 50 uL of solution 1 (individually, A-G) was placed onto a glass slide. Solution 2 (individually, A-G) was added to a second 3 mL syringe (Becton-Dickinson), and the syringe was attached to a second microcatheter. Holding the end of the catheter above the first solution on the glass slide, and applying moderate pressure to the syringe, approximately 50 uL of solution 2 was added to solution 1 on the glass slide.

After mixing for 2-5 seconds, the mixture polymerized and formed a semi-firm gel having elastomeric properties.

**Table 2**

| | MD-A (reduct.) | MD-A (oxidant) | Microcatheter diameter | Viscosity (approx.) | Flow rate (approx.) | Matrix properties |
|---|---|---|---|---|---|---|
| A | 250 mg/mL | 250 mg/mL | 1.7 fr | 35 cP | 40-50 uL/min | Semi-firm gel |
| B | 500 mg/mL | 500 mg/mL | 1.7 fr | 75 cP | 15-17 uL/min | Semi-firm gel |
| | MD-MA (reduct.) | MD-MA (oxidant) | | | | |
| C | 250 mg/mL | 250 mg/mL | 1.7 fr | 36 | 40-50 uL/min | Semi-firm gel |
| D | 350 mg/mL | 350 mg/mL | 1.7 Fr | 45 | 35-40 uL/min | Semi-firm gel |
| E | 500 mg/mL | 500 mg/mL | 1.7 Fr | 78 | 15-17 uL/min | Semi-firm gel |
| F | 250 mg/mL | 250 mg/mL | 2.5 Fr | 36 | 60-70 uL/min | Semi-firm gel |
| G | 500 mg/mL | 500 mg/mL | 2.5 fr | 78 | 25-30 uL/min | Semi-firm gel |

### Example 25

### Formation of Polvalditol-acrylate Biodegradable Matrix Using REDOX Chemistry

Reductant and oxidant solutions including Polyalditol-acrylate (PD-A) were prepared (see Table 3). Oxidant solutions were prepared as follows: 500 mg of PD-A (as prepared in Example 13) were individually placed in an 8 mL vial. To the PD-A was added various amounts of ammonium persulfate (Sigma) (see Table 3, rows A-H), potassium persulfate (see Table 3, rows M-P) or sodium persulfate (see Table 3, rows I-L) and 1,000 uL PBS. Reductant solutions were prepared as follows: 500 mg of PD-A was placed in a second 8 mL vial. To this PD-A was added either 20 uL or 40 uL TEMED, 40 uL 1N hydrochloric acid (VWR) and 960 uL Phosphate Buffered Saline (Sigma; pH 7.4).

Each polymerization experiment was performed as follows: 50 uL of oxidant solution was transferred to a glass slide; subsequently 50 uL of reductant solution was added to the oxidant solution. After mixing for 5 seconds at 23°C or 37°C, the mixture polymerized and formed gels having elastomeric properties.

**Table 3**

| | *PD-A* | *Oxidant* | *Oxidant* *Conc* *(mg*/*ml)* | *TEMED* *(ul*/*ml)* | *Temperature* *(Celsius)* | *Crosslink time* *(secs)* | *Matrix* *properties* |
|---|---|---|---|---|---|---|---|
| A | 500 mg/mL | Ammonium Persulfate | 10 | 20 UL | 23 | 240 s | Semi-firm gel |
| B | 500 mg/mL | Ammonium Persulfate | 15 | 20 | 23 | 120 s | Semi-firm gel |
| C | 500 mg/mL | Ammonium Persulfate | 50 | 20 | 23 | 60 s | Semi-firm gel |
| D | 500 mg/mL | Ammonium Persulfate | 100 | 20 | 23 | 45 s | Semi-firm gel |
| E | 500 mg/mL | Ammonium Persulfate | 10 | 40 | 23 | 120 s | Semi-firm gel |
| F | 500 mg/mL | Ammonium Persulfate | 50 | 40 | 23 | 50 s | Semi-firm gel |
| G | 500 mg/mL | Ammonium Persulfate | 15 | 20 | 37 | 60 s | Semi-firm gel |
| H | 500 mg/mL | Ammonium Persulfate | 50 | 20 | 37 | 20 s | Semi-firm gel |
| I | 500 mg/mL | Sodium Persulfate | 5 | 20 | 23 | 600 s | Soft gel |
| J | 500 mg/mL | Sodium Persulfate | 10 | 20 | 23 | 360 s | Soft gel |
| K | 500 mg/mL | Sodium Persulfate | 5 | 20 | 37 | 90 s | Soft gel |
| L | 500 mg/mL | Sodium Persulfate | 10 | 20 | 37 | 90 s | Semi-firm gel |
| M | 500 mg/mL | Potassium Persulfate | 30 | 20 | 23 | 240 s | Semi-firm gel |
| N | 500mg/mL | Potassium Persulfate | 30 | 40 | 23 | 90s s | Semi-firm gel |
| O | 500 mg/mL | Potassium Persulfate | 30 | 20 | 37 | 75 s | Semi-firm gel |
| P | 500mg/mL | Potassium Persulfate | 30 | 40 | 37 | 30 s | Semi-firm gel |

### Example 26

### Cell Viability Within Polyalditol-Acrylate REDOX Components

Solutions were prepared having the concentrations indicated in Table 4.

Cell suspensions were prepared as follows: PC-12 cells (ATCC; passage 5; 75% confluency) were harvested from a T-75 flask using Trypsin-EDTA for 2 minutes. The cells were placed in a 15 mL polyethylene conical (VWR) and centrifuged in F-12k media without serum for 4 minutes at 500 rpm. The cells were counted using a hemocytometer, centrifuged for 4 minutes at 500 rpm, and resuspended at 600,000 cells/mL in sterile PBS.

In order to determine the effect of individual components of the matrix forming compositions on cell viability, solutions A-G were independently mixed with PC-12 cells. 50 uL of cell suspension was added to 350 uL of solutions A-G (Table 4). After a 15 minute incubation, cell viability was assessed using a Live/Dead^{™} Viability/Cytotoxicity Kit (cat.# L3224; Molecular Probes, Eugene, OR).

The effect of a formed matrix on cell viability was also tested. To form the PD-A matrix, solution #1 (200 mg PD-A, 10 uL TEMED, 20 uL 1N HCl, 470 uL PBS) was added in the amount of 200 uL to a 1.6 ml eppendorf (VWR). Solution #2 (400 mg PD-A, 10 mg Potassium persulfate, 75K PC-12 cells, 500 uL PBS) was added in the amount of 200 uL to solution #1 in the eppendorf and mixed for 10 seconds. After a 15 minute incubation, cell viability was assessed using the Live/Dead^{™} Viability/Cytotoxicity Kit.

**Table 4**

| | *Component(s)* | *Concentration* *(in PBS)* | *Incubation* *time* | *Cell viability (%)* |
|---|---|---|---|---|
| A | TEMED/PBS | 0.2% (V/V) | 15 min | 10-30% |
| B | Sodium persulfate | 5 mg/ml | 15 min | 90% |
| C | Potassium persulfate | 10 mg/ml | 15 min | 90% |
| D | Ammonium persulfate | 10 mg/ml | 15 min | 90% |
| E | Ammonium Persulfate | 120 mg/ml | 15 min | 90% |
| F | PD-A | 400 mg/ml | 15 min | 90% |
| G | PD-A + TEMED | 400mg/ml +0.2% (v/v) | 15 min. | 50% |
| H | PD-A matrix | | 15 min | 80% |
| I | PBS | | 15 min | 90% |

## Claims

1. A composition comprising natural biodegradable polysaccharide comprising pendent polymerisable groups and a first member of a redox pair,
for use in a method of medical treatment which comprises forming a biodegradable occlusion at a target site within the body by delivering the composition to the target site and contacting the composition at the target site with a second member of the redox pair where the redox pair initiates reaction of the pendent polymerisable groups of the natural biodegradable polysaccharide to form the biodegradable occlusion.

2. A composition of claim 1 for use in a said method of treatment in which the composition is delivered to the target site via a delivery conduit.

3. A composition of claim 2 in which the delivery conduit comprises a microcatheter having a diameter of 0.77 mm (2.3 Fr) or less.

4. A composition of any one of claims 1 to 3 having a viscosity less than 45 mPa·s.

5. A composition of any one of the preceding claims for use in a said method wherein the target site is an aneurysm.

6. A composition of any one of the preceding claims for use in a said method wherein for said contacting an article is inserted into the target site, and the article is associated with the second member of the redox pair.

7. A composition of claim 6 wherein said article is an aneurysm coil, wire or string.

8. A composition of claim 1, 6 or 7 wherein said first member of a redox pair is a reducing agent.

9. A composition of claim 1 for use in a said method wherein said contacting comprises delivering to the target site a second composition that includes the second member of the redox pair.

10. A composition of claim 9 for use in a said method in which said second composition comprises natural biodegradable polysaccharide comprising pendent polymerisable groups and said second member of the redox pair.

11. A composition of any one of the preceding claims wherein the natural biodegradable polysaccharide has a molecular weight of 100,000 Da or less.

12. A composition of claim 11 wherein the natural biodegradable polysaccharide has a molecular weight of 50,000 Da or less.

13. A composition of claim 12 wherein the natural biodegradable polysaccharide has a molecular weight in the range of 1000 Da to 10,000 Da.

14. A composition according to any one of the preceding claims except claim 8 wherein the natural biodegradable polysaccharide is selected from the group consisting of amylose, maltodextrin, cyclodextrin and polyalditol.

15. A composition according to any one of the preceding claims except claim 8 wherein the biodegradable polysaccharide comprises a non-reducing natural biodegradable polysaccharide.

16. A composition of claim 1 or 15 wherein the biodegradable polysaccharide is polyalditol.

17. A composition of any one of the preceding claims except claim 8 which comprises a pro-fibrotic agent.

18. A composition of claim 17 wherein the pro-fibrotic agent comprises collagen or an active domain thereof.

19. A composition of claim 18 wherein the collagen is collagen I or an active domain thereof.

20. A composition of claim 17 wherein the pro-fibrotic agent comprises a polymerisable group.

21. A kit for forming a biodegradable occlusion at a target site within the body, comprising
a first composition for delivery to the target site, comprising natural biodegradable polysaccharide comprising pendent polymerisable groups and a first member of a redox pair, and
a second composition for delivery to the target site, comprising natural biodegradable polysaccharide comprising pendent polymerisable groups and the second member of the redox pair,
whereby in use, on contacting the first and second compositions, the resulting redox pair initiates polymerisation of the polymerisable groups to form the biodegradable occlusion.

22. A kit for forming a biodegradable occlusion at a target site within a body, the kit comprising:
a natural biodegradable polysaccharide comprising a pendent polymerisable group;
a first member of a redox pair;
an article configured to be delivered to the target site, and
a second member of the redox pair.

23. A kit of claim 22 comprising a first composition comprising the natural biodegradable polysaccharide and the first member of the redox pair.

24. A kit of claim 22 or 23 wherein said article comprises a neuroaneurysm coil.

25. A kit of claim 22, 23 or 24 wherein the second member of the redox pair is associated with said article.

## Patentansprüche

**1.** Zusammensetzung, umfassend natürliches, biologisch abbaubares Polysaccharid mit polymerisierbaren seitenständigen Gruppen und einem ersten Element eines Redoxpaars, zur Verwendung in einem Verfahren zur medizinischen Behandlung, das das Ausbilden eines biologisch abbaubaren Einschlusses an einer Zielstelle innerhalb des Körpers durch das Zuführen der Zusammensetzung an die Zielstelle und das Kontaktieren der Zusammensetzung an der Zielstelle mit einem zweiten Element des Redoxpaars umfasst, wobei das Redoxpaar das Umsetzen der polymerisierbaren seitenständigen Gruppen des natürlichen, biologisch abbaubaren Polysaccharids zur Ausbildung des biologisch abbaubaren Einschlusses zu initiieren.

**2.** Zusammensetzung nach Anspruch 1 zur Verwendung in dem Behandlungsverfahren, im Zuge dessen die Zusammensetzung über eine Zufuhrleitung (???) an die Zielstelle zugeführt wird.

**3.** Zusammensetzung nach Anspruch 2, worin die Zufuhrleitung einen Mikrokatheter mit einem Durchmesser von 0,77 mm (2,3 Fr) oder weniger umfasst.

**4.** Zusammensetzung nach einem der Ansprüche 1 bis 3 mit einer Viskosität von weniger als 45 mPa·s.

**5.** Zusammensetzung nach einem der vorangegangenen Ansprüche zur Verwendung in dem Verfahren, wobei die Zielstelle ein Aneurysma ist.

**6.** Zusammensetzung nach einem der vorangegangenen Ansprüche zur Verwendung in dem Verfahren, wobei zum Kontaktieren ein Gegenstand in die Zielstelle eingeführt wird und der Gegenstand mit dem zweiten Element des Redoxpaars verbunden ist.

**7.** Zusammensetzung nach Anspruch 6, wobei es sich bei dem Gegenstand um eine Aneurysmaspule, einen Draht oder eine Schnur handelt.

**8.** Zusammensetzung nach Anspruch 1, 6 oder 7, worin das erste Element des Redoxpaars ein Reduktionsmittel ist.

**9.** Zusammensetzung nach Anspruch 1 zur Verwendung in einem solchen Verfahren, wobei das Kontaktieren das Zuführen einer zweiten Zusammensetzung, die das zweite Element eines Redoxpaars umfasst, an die Zielstelle umfasst.

**10.** Zusammensetzung nach Anspruch 9 zur Verwendung in einem solchen Verfahren, wobei die zweite Zusammensetzung natürliches, biologisch abbaubares Polysaccharid umfasst, das polymerisierbare seitenständige Gruppen und das zweite Element des Redoxpaars umfasst.

**11.** Zusammensetzung nach einem der vorangegangenen Ansprüche, worin das natürliche, biologisch abbaubare Polysaccharid ein Molekulargewicht von 100.000 Da oder weniger aufweist.

**12.** Zusammensetzung nach Anspruch 11, worin das natürliche, biologisch abbaubare Polysaccharid ein Molekulargewicht von 50.000 Da oder weniger aufweist.

**13.** Zusammensetzung nach Anspruch 12, worin das natürliche, biologisch abbaubare Polysaccharid ein Molekulargewicht im Bereich von 10.000 bis 10.000 Da aufweist.

**14.** Zusammensetzung nach einem der vorangegangenen Ansprüche mit Ausnahme von Anspruch 8, worin das natürliche, biologisch abbaubare Polysaccharid aus der aus Amylose, Maltodextrin, Cyclodextrin und Polyalditol bestehenden Gruppe ausgewählt ist.

**15.** Zusammensetzung nach einem der vorangegangenen Ansprüche mit Ausnahme von Anspruch 8, worin des natürliche, biologisch abbaubare Polysaccharid ein nicht-reduzierendes, natürliches, biologisch abbaubares Polysaccharid umfasst.

**16.** Zusammensetzung nach Anspruch 1 oder 15, worin das biologisch abbaubare Polysaccharid Polyalditol ist.

**17.** Zusammensetzung nach einem der vorangegangenen Ansprüche mit Ausnahme von Anspruch 8, die ein profibrotisches (???) Mittel umfasst.

**18.** Zusammensetzung nach Anspruch 17, worin das profibrotische Mittel Collagen oder eine aktive Domäne davon umfasst.

**19.** Zusammensetzung nach Anspruch 18, worin das Collagen Collagen I oder eine aktive Domäne davon ist.

**20.** Zusammensetzung nach Anspruch 17, worin das profibrotische Mittel eine polymerisierbare Gruppe umfasst.

**21.** Set zur Ausbildung eines biologisch abbaubaren Einschlusses an einer Zielstelle im Körper, umfassend:
eine erste Zusammensetzung, die ein natürliches, biologisch abbaubares Polysaccharid umfasst, das polymerisierbare seitenständige Gruppen und ein erstes Element eines Redoxpaars umfasst, zur Zufuhr an die Zielstelle und
eine zweite Zusammensetzung, die ein natürliches, biologisch abbaubares Polysaccharid umfasst, das polymerisierbare seitenständige Gruppen und das zweite Element des Redoxpaars umfasst, zur Zufuhr an die Zielstelle,
wobei bei Verwendung bei Kontaktieren der ersten und der zweiten Zusammensetzung das resultierende Redoxpaar die Polymerisierung der polymerisierbaern Gruppen initiiert, um den biologisch abbaubaren Einschluss auszubilden.

**22.** Set zur Ausbildung eines biologisch abbaubaren Einschlusses an einer Zielstelle im Körper, wobei das Set Folgendes umfasst:
ein natürliches, biologisch abbaubares Polysaccharid, das eine polymerisierbare seitenständige Gruppe umfasst;
ein erstes Element eines Redoxpaars;
einen für die Zufuhr an die Zielstelle konfigurierten Gegenstand und
das zweite Element des Redoxpaars.

**23.** Set nach Anspruch 22, umfassend eine erste Zusammensetzung, die das natürliche, biologisch abbaubare Polysaccharid und das erste Element des Redoxpaars umfasst.

**24.** Set nach Anspruch 22 oder 23, worin der Gegenstand eine Neuroaneurysmaspule umfasst.

**25.** Set nach Anspruch 22, 23 oder 24, worin das zweite Element des Redoxpaars mit dem Gegenstand verbunden ist.

## Revendications

1. Composition comprenant des polysaccharides biodégradables naturels comprenant des groupes polymérisables pendants et un premier membre d'une paire redox,
pour utilisation dans une méthode de traitement médicale qui comprend la formation d'une occlusion biodégradable à un site cible dans le corps en délivrant la composition au site cible et en mettant en contact la composition au site cible avec un deuxième membre de la paire redox, où la paire redox initie la réaction des groupes polymérisables pendants du polysaccharide biodégradable naturel pour former l'occlusion biodégradable.

2. Composition selon la revendication 1, pour utilisation dans ladite méthode de traitement précitée dans laquelle la composition est délivrée au site cible par une conduite de délivrance.

3. Composition selon la revendication 2, dans laquelle la conduite de délivrance comprend un microcathéter d'un diamètre de 0,77 mm (2,3 Fr) ou moins.

4. Composition selon l'une quelconque des revendications 1 à 3, ayant une viscosité inférieure à 45 mPa.s.

5. Composition selon l'une quelconque des revendications précédentes pour utilisation dans ladite méthode précitée, où le site cible est un anévrisme.

6. Composition selon l'une quelconque des revendications précédentes pour utilisation dans ladite méthode précitée, dans laquelle pour ladite mise en contact, un article est inséré dans le site cible, et l'article est associé au deuxième élément de la paire redox.

7. Composition selon la revendication 6, dans laquelle ledit article est une spire, fil ou cordon d'anevrisme.

8. Composition selon la revendication 1, 6 ou 7, dans laquelle ledit premier élément d'une paire redox est un agent réducteur.

9. Composition selon la revendication 1 pour utilisation dans ladite méthode précitée, dans laquelle ladite mise en contact comprend la délivrance au site cible d'une deuxième composition qui comprend le deuxième élément de la paire redox.

10. Composition selon la revendication 9 pour utilisation dans ladite méthode précitée dans laquelle ladite deuxième composition comprend du polysaccharide biodégradable naturel comprenant des groupes polymérisables pendants et ledit deuxième élément de la paire redox.

11. Composition selon l'une quelconque des revendications précédentes, dans laquelle le polysaccharide biodégradable naturel a un poids moléculaire de 100 000 Da ou moins.

12. Composition selon la revendication 11, dans laquelle le polysaccharide biodégradable naturel a un poids moléculaire de 50 000 Da ou moins.

13. Composition selon la revendication 12, dans laquelle le polysaccharide biodégradable naturel a un poids moléculaire dans la plage de 1000 Da à 10 000 Da.

14. Composition selon l'une quelconque des revendications précédentes, exceptée la revendication 8, dans laquelle le polysaccharide biodégradable naturel est sélectionné dans le groupe consistant en amylose, maltodextrine, cyclodextrine et polyalditol.

15. Composition selon l'une quelconque des revendications précédentes, exceptée la revendication 8, dans laquelle le polysaccharide biodégradable comprend un polysaccharide biodégradable naturel non-réducteur.

16. Composition selon la revendication 1 ou 15, dans laquelle le polysaccharide biodégradable est le polyalditol.

17. Composition selon l'une quelconque des revendications précédentes, exceptée la revendication 8, qui comprend un agent pro-fibrotique.

18. Composition selon la revendication 17, dans laquelle l'agent pro-fibrotique comprend du collagène ou un domaine actif de celui-ci.

19. Composition selon la revendication 18, dans laquelle le collagène est le collagène I ou un domaine actif de celui-ci.

20. Composition selon la revendication 17, dans laquelle l'agent pro-fibrotique comprend un groupe polymérisable.

21. Kit pour former une occlusion biodégradable à un site cible dans le corps, comprenant
une première composition pour la délivrance au site cible, comprenant un polysaccharide biodégradable naturel comprenant des groupes polymérisables pendants et un premier membre d'une paire redox, et
une deuxième composition pour la délivrance au site cible, comprenant un polysaccharide biodégradable naturel comprenant des groupes polymérisables pendants et le deuxième membre de la paire redox,
par quoi en utilisation, lors de la mise en contact des première et deuxième compositions, la paire redox obtenue initie la polymérisation des groupes polymérisables pour former l'occlusion biodégradable.

22. Kit pour former une occlusion biodégradable à un site cible dans le corps, le kit comprenant:
un polysaccharide biodégradable naturel comprenant un groupe polymérisable pendant;
un premier membre d'une paire rédox;
un article configuré pour être délivré au site cible, et
un deuxième membre de la paire redox.

23. Kit selon la revendication 22, comprenant une première composition comprenant le polysaccharide biodégradable naturel et le premier membre de la paire redox.

24. Kit selon la revendication 22 ou 23, dans lequel ledit article comprend une spire de neuroanévrisme.

25. Kit selon la revendication 22, 23 ou 24, dans lequel le deuxième membre de la paire redox est associé audit article.
